# EUROPEAN PATENT APPLICATION

(11) **EP 1 347 053 A1**
(43) Date of publication of application: **24.09.2003**
(21) Application number: 01999253.6
(22) Date of filing: 06.12.2001
(51) Int. Cl.: C12N 15/12, C12P 21/02, C07K 14/705, C12N 1/15, C12N 1/19, C12N 1/21, C12N 5/10

(54) **PROCESS FOR PRODUCING RFRP**

(30) Priority: 07.12.2000 JP 2000373125
(71) Applicant: Takeda Chemical Industries, Ltd., Osaka-shi, Osaka 541-0045 (JP)
(72) Inventor: SUENAGA, Masato, Hikari-shi, Yamaguchi 743-0075 (JP); YAMADA, Takao, Matsubara-shi, Osaka 580-0003 (JP); NISHIMURA, Osamu, Kawanishi-shi, Hyogo 666-0112 (JP)
(74) Representative: Lewin, John Harvey
(86) International application number: JP0110668
(87) International publication number: WO02046405

(57) **Abstract**

The present invention is intended to provide an advantageous method of industrially producing RFamide peptides in a large scale. The present invention provides the method of producing RFamide peptides, which comprises subjecting a fusion protein or peptide, in which an RFamide peptide is ligated to the N-terminal of a protein or peptide having a cysteine at the N-terminal, to the reaction for cleavage of a peptide bond on the amino acid side of the cysteine residue.

[SEQUENCE LISTING]

<110> Takeda Chemical Industries, Ltd.

<120> Method of Production for RFRP

<130> P01-0262PCT

<140> PCT/P01/10668
<141> 2001-12-06

<150> JP 2000-373125
<151> 2000-12-07

<160> 30

<210> 1
<211> 9
<212> PRT
<213> Human

<400> 1
Ser Phe Ala Asn Leu Pro Leu Arg Phe
1               5               9

<210> 2
<211> 27
<212> DNA
<213> Human

<400> 2
agctttgcga atctgccgct gcgtttt          27

<210> 3
<211> 12
<212> PRT

<213> Human

<400> 3
Met Pro His Ser Phe Ala Asn Leu Pro Leu Arg Phe
1               5                   10      12

<210> 4
<211> 36
<212> DNA
<213> Human

<400> 4
atgccgcata gctttgcgaa tctgccgctg cgtttt      36

<210> 5
<211> 20
<212> PRT
<213> Human

<400> 5
Met Ser Thr Pro Ala Val Asn Lys Met Pro His Ser Phe Ala Asn Leu
1               5                   10                  15
Pro Leu Arg Phe
            20

<210> 6
<211> 60
<212> DNA
<213> Human

<400> 6
atgagcaccc cggcggtgaa taaaatgccg catagctttg cgaatctgcc gctgcgtttt   60

<210> 7
<211> 37
<212> PRT

<213> Human

<400> 7
Ser Leu Asn Phe Glu Glu Leu Lys Asp Trp Gly Pro Lys Asn Val Ile
1               5                   10                  15
Lys Met Ser Thr Pro Ala Val Asn Lys Met Pro His Ser Phe Ala Asn
            20                  25                  30
Leu Pro Leu Arg Phe
        35      37

<210> 8
<211> 111
<212> DNA
<213> Human

<400> 8
agcctgaact ttgaagaact gaaagattgg ggtccgaaaa atgtgattaa aatgagcacc   60
ccggcggtga ataaaatgcc gcatagcttt gcgaatctgc cgctgcgttt t           111

<210> 9
<211> 8
<212> PRT
<213> Human

<400> 9
Val Pro Asn Leu Pro Gln Arg Phe
1               5           8

<210> 10
<211> 24
<212> DNA
<213> Human

<400> 10
gttcctaacc tgccccaaag gttt                                          24

<210> 11
<211> 17
<212> PRT
<213> Human

<400> 11
Asn Met Glu Val Ser Leu Val Arg Arg Val Pro Asn Leu Pro Gln Arg
1               5                   10                  15
Phe
17

<210> 12
<211> 51
<212> DNA
<213> Human

<400> 12
aatatggagg tgagcctcgt gagacgtgtt cctaacctgc cccaaaggtt t          51

<210> 13
<211> 146
<212> PRT
<213> Human

<400> 13
Pro Ala Leu Pro Glu Asp Gly Gly Ser Gly Ala Phe Pro Pro Gly His
1               5                   10                  15
Phe Lys Asp Pro Lys Arg Leu Tyr Cys Lys Asn Gly Gly Phe Phe Leu
            20                  25                  30
Arg Ile His Pro Asp Gly Arg Val Asp Gly Val Arg Glu Lys Ser Asp
        35                  40                  45
Pro His Ile Lys Leu Gln Leu Gln Ala Glu Glu Arg Gly Val Val Ser
    50                  55                  60
Ile Lys Gly Val Ser Ala Asn Arg Tyr Leu Ala Met Lys Glu Asp Gly
65                  70                  75                  80
Arg Leu Leu Ala Ser Lys Ser Val Thr Asp Glu Cys Phe Phe Phe Glu

85                    90                    95
Arg Leu Glu Ser Asn Asn Tyr Asn Thr Tyr Arg Ser Arg Lys Tyr Thr
            100                   105                   110
Ser Trp Tyr Val Ala Leu Lys Arg Thr Gly Gln Tyr Lys Leu Gly Ser
        115                   120                   125
Lys Thr Gly Pro Gly Gln Lys Ala Ile Leu Phe Leu Pro Met Ser Ala
    130                   135                   140
Lys Ser
145 146

<210> 14
<211> 432
<212> DNA
<213> Human

<400> 14
cccgaggatg gcggcagcgg cgccttcccg cccggccact tcaaggaccc caagcggctg    60
tactgcaaaa acgggggctt cttcctgcgc atccaccccg acggccgagt tgacggggtc   120
cgggagaaga gcgaccctca catcaagcta caacttcaag cagaagagag aggagttgtg   180
tctatcaaag gagtgagcgc taatcgttac ctggctatga aggaagatgg aagattacta   240
gcttctaagt ctgttacgga tgagtgtttc ttttttgaac gattggaatc taataactac   300
aatacttacc ggtcaaggaa atacaccagt tggtatgtgg cactgaaacg aactgggcag   360
tataaacttg gatccaaaac aggacctggg cagaaagcta tactttttct tccaatgtct   420
gctaagagct gc                                                       432

<210> 15
<211> 58
<212> DNA
<213> Artificial Sequence

<220>
<223> Primer

<400> 15
tatgagcctg aactttgaag aactgaaaga ttggggtccg aaaaatgtga ttaaaatg    58

<210> 16
<211> 61
<212> DNA
<213> Artificial Sequence

<220>
<223> Primer

<400> 16
agcaccccgg cggtgaataa aatgccgcat agctttgcga atctgccgct gcgtttttgc     60
c                                                                     61

<210> 17
<211> 62
<212> DNA
<213> Artificial Sequence

<220>
<223> Primer

<400> 17
ggtgctcatt ttaatcacat ttttcggacc ccaatctttc agttcttcaa agttcaggct     60
ca                                                                    62

<210> 18
<211> 59
<212> DNA
<213> Artificial Sequence

<220>
<223> Primer

<400> 18
tcggggcaaa aacgcagcgg cagattcgca aagctatgcg gcattttatt caccgccgg     59

<210> 19

<211> 180
<212> PRT
<213> Human

<400> 19
Met Glu Ile Ile Ser Ser Lys Leu Phe Ile Leu Leu Thr Leu Ala Thr
1               5                   10                  15
Ser Ser Leu Leu Thr Ser Asn Ile Phe Cys Ala Asp Glu Leu Val Met
            20                  25                  30
Ser Asn Leu His Ser Lys Glu Asn Tyr Asp Lys Tyr Ser Glu Pro Arg
        35                  40                  45
Gly Tyr Pro Lys Gly Glu Arg Ser Leu Asn Phe Glu Glu Leu Lys Asp
    50                  55                  60
Trp Gly Pro Lys Asn Val Ile Lys Met Ser Thr Pro Ala Val Asn Lys
65                  70                  75                  80
Met Pro His Ser Phe Ala Asn Leu Pro Leu Arg Phe Gly Arg Asn Val
                85                  90                  95
Gln Glu Glu Arg Ser Ala Gly Ala Thr Ala Asn Leu Pro Leu Arg Ser
            100                 105                 110
Gly Arg Asn Met Glu Val Ser Leu Val Arg Arg Val Pro Asn Leu Pro
        115                 120                 125
Gln Arg Phe Gly Arg Thr Thr Thr Ala Lys Ser Val Cys Arg Met Leu
    130                 135                 140
Ser Asp Leu Cys Gln Gly Ser Met His Ser Pro Cys Ala Asn Asp Leu
145                 150                 155                 160
Phe Tyr Ser Met Thr Cys Gln His Gln Glu Ile Gln Asn Pro Asp Gln
                165                 170                 175
Lys Gln Ser Arg
            180

<210> 20
<211> 540
<212> DNA
<213> Human

<400> 20

atggaaatta tttcatcaaa actattcatt ttattgactt tagccacttc aagcttgtta 60
acatcaaaca ttttttgtgc agatgaatta gtgatgtcca atcttcacag caaagaaaat 120
tatgacaaat attctgagcc tagaggatac ccaaaagggg aaagaagcct caattttgag 180
gaattaaaag attggggacc aaaaaatgtt attaagatga gtacacctgc agtcaataaa 240
atgccacact ccttcgccaa cttgccattg agatttggga ggaacgttca agaagaaaga 300
agtgctggag caacagccaa cctgcctctg agatctgga agaaatatgga ggtgagcctc 360
gtgagacgtg ttcctaacct gccccaaagg tttgggagaa caacaacagc caaaagtgtc 420
tgcaggatgc tgagtgattt gtgtcaagga tccatgcatt caccatgtgc caatgactta 480
ttttactcca tgacctgcca gcaccaagaa atccagaatc ccgatcaaaa acagtcaagg 540

<210> 21
<211> 196
<212> PRT
<213> Human

<400> 21
Met Glu Ile Ile Ser Ser Lys Leu Phe Ile Leu Leu Thr Leu Ala Thr
1               5                   10                  15
Ser Ser Leu Leu Thr Ser Asn Ile Phe Cys Ala Asp Glu Leu Val Met
            20                  25                  30
Ser Asn Leu His Ser Lys Glu Asn Tyr Asp Lys Tyr Ser Glu Pro Arg
        35                  40                  45
Gly Tyr Pro Lys Gly Glu Arg Ser Leu Asn Phe Glu Glu Leu Lys Asp
    50                  55                  60
Trp Gly Pro Lys Asn Val Ile Lys Met Ser Thr Pro Ala Val Asn Lys
65                  70                  75                  80
Met Pro His Ser Phe Ala Asn Leu Pro Leu Arg Phe Gly Arg Asn Val
                85                  90                  95
Gln Glu Glu Arg Ser Ala Gly Ala Thr Ala Asn Leu Pro Leu Arg Ser
            100                 105                 110
Gly Arg Asn Met Glu Val Ser Leu Val Arg Arg Val Pro Asn Leu Pro
        115                 120                 125
Gln Arg Phe Gly Arg Thr Thr Thr Ala Lys Ser Val Cys Arg Met Leu
    130                 135                 140
Ser Asp Leu Cys Gln Gly Ser Met His Ser Pro Cys Ala Asn Asp Leu
145                 150                 155                 160

Phe Tyr Ser Met Thr Cys Gln His Gln Glu Ile Gln Asn Pro Asp Gln
                165                 170                 175
Lys Gln Ser Arg Arg Leu Leu Phe Lys Lys Ile Asp Asp Ala Glu Leu
            180                 185                 190
Lys Gln Glu Lys
        195

<210> 22
<211> 588
<212> DNA
<213> Human

<400> 22
atggaaatta tttcatcaaa actattcatt ttattgactt tagccacttc aagcttgtta    60
acatcaaaca ttttttgtgc agatgaatta gtgatgtcca atcttcacag caaagaaaat   120
tatgacaaat attctgagcc tagaggatac ccaaaagggg aaagaagcct caattttgag   180
gaattaaaag attggggacc aaaaaatgtt attaagatga gtacacctgc agtcaataaa   240
atgccacact ccttcgccaa cttgccattg agatttggga ggaacgttca agaagaaaga   300
agtgctggag caacagccaa cctgcctctg agatctggaa gaaatatgga ggtgagcctc   360
gtgagacgtg ttcctaacct gccccaaagg tttgggagaa caacaacagc caaaagtgtc   420
tgcaggatgc tgagtgattt gtgtcaagga tccatgcatt caccatgtgc caatgactta   480
ttttactcca tgacctgcca gcaccaagaa atccagaatc ccgatcaaaa acagtcaagg   540
agactgctat tcaagaaaat agatgatgca gaattgaaac aagaaaaa                588

<210> 23
<211> 196
<212> PRT
<213> Bovine

<400> 23
Met Glu Ile Ile Ser Leu Lys Arg Phe Ile Leu Leu Met Leu Ala Thr
 1               5                  10                  15
Ser Ser Leu Leu Thr Ser Asn Ile Phe Cys Thr Asp Glu Ser Arg Met
            20                  25                  30
Pro Asn Leu Tyr Ser Lys Lys Asn Tyr Asp Lys Tyr Ser Glu Pro Arg
        35                  40                  45

Gly Asp Leu Gly Trp Glu Lys Glu Arg Ser Leu Thr Phe Glu Glu Val
    50                  55                  60
Lys Asp Trp Ala Pro Lys Ile Lys Met Asn Lys Pro Val Val Asn Lys
65                  70                  75                  80
Met Pro Pro Ser Ala Ala Asn Leu Pro Leu Arg Phe Gly Arg Asn Met
                85                  90                  95
Glu Glu Glu Arg Ser Thr Arg Ala Met Ala His Leu Pro Leu Arg Leu
            100                 105                 110
Gly Lys Asn Arg Glu Asp Ser Leu Ser Arg Trp Val Pro Asn Leu Pro
        115                 120                 125
Gln Arg Phe Gly Arg Thr Thr Thr Ala Lys Ser Ile Thr Lys Thr Leu
    130                 135                 140
Ser Asn Leu Leu Gln Gln Ser Met His Ser Pro Ser Thr Asn Gly Leu
145                 150                 155                 160
Leu Tyr Ser Met Ala Cys Gln Pro Gln Glu Ile Gln Asn Pro Gly Gln
                165                 170                 175
Lys Asn Leu Arg Arg Arg Gly Phe Gln Lys Ile Asp Asp Ala Glu Leu
            180                 185                 190
Lys Gln Glu Lys
        195

<210> 24
<211> 588
<212> DNA
<213> Bovine

<400> 24
atggaaatta tttcattaaa acgattcatt ttattgatgt tagccacttc aagcttgtta    60
acatcaaaca tcttctgcac agacgaatca aggatgccca atctttacag caaaaagaat   120
tatgacaaat attccgagcc tagaggagat ctaggctggg agaaagaaag aagtcttact   180
tttgaagaag taaaagattg ggctccaaaa attaagatga ataaacctgt agtcaacaaa   240
atgccacctt ctgcagccaa cctgccactg agatttggga ggaacatgga agaagaaagg   300
agcactaggg cgatggccca cctgcctctg agactcggaa aaaatagaga ggacagcctc   360
tccagatggg tcccaaatct gccccagagg tttggaagaa caacaacagc caaaagcatt   420
accaagaccc tgagtaattt gctccagcag tccatgcatt caccatctac caatgggcta   480
ctctactcca tggcctgcca gccccaagaa atccagaatc ctggtcaaaa gaacctaagg   540

agacggggat tccagaaaat agatgatgca gaattgaaac aagaaaaa 588

<210> 25
<211> 188
<212> PRT
<213> Mouse

<400> 25
Met Glu Ile Ile Ser Leu Lys Arg Phe Ile Leu Leu Thr Val Ala Thr
1               5                   10                  15
Ser Ser Phe Leu Thr Ser Asn Thr Phe Cys Thr Asp Glu Phe Met Met
            20                  25                  30
Pro His Phe His Ser Lys Glu Gly Asp Gly Lys Tyr Ser Gln Leu Arg
        35                  40                  45
Gly Ile Pro Lys Gly Glu Lys Glu Arg Ser Val Ser Phe Gln Glu Leu
    50                  55                  60
Lys Asp Trp Gly Ala Lys Asn Val Ile Lys Met Ser Pro Ala Pro Ala
65                  70                  75                  80
Asn Lys Val Pro His Ser Ala Ala Asn Leu Pro Leu Arg Phe Gly Arg
                85                  90                  95
Thr Ile Asp Glu Lys Arg Ser Pro Ala Ala Arg Val Asn Met Glu Ala
            100                 105                 110
Gly Thr Arg Ser His Phe Pro Ser Leu Pro Gln Arg Phe Gly Arg Thr
        115                 120                 125
Thr Ala Arg Ser Pro Lys Thr Pro Ala Asp Leu Pro Gln Lys Pro Leu
    130                 135                 140
His Ser Leu Gly Ser Ser Glu Leu Leu Tyr Val Met Ile Cys Gln His
145                 150                 155                 160
Gln Glu Ile Gln Ser Pro Gly Gly Lys Arg Thr Arg Arg Gly Ala Phe
                165                 170                 175
Val Glu Thr Asp Asp Ala Glu Arg Lys Pro Glu Lys
            180                 185

<210> 26
<211> 564
<212> DNA

<213> Mouse

<400> 26
atggaaatta tttcattaaa acgattcatt ttattgactg tggcaacttc aagcttctta 60
acatcaaaca ccttctgtac agatgagttc atgatgcctc attttcacag caaagaaggt 120
gacggaaaat actcccagct gagaggaatc ccaaaagggg aaaaggaaag aagtgtcagt 180
tttcaagaac taaaagattg gggggcaaag aatgttatta agatgagtcc agcccctgcc 240
aacaaagtgc cccactcagc agccaacctg cccctgagat ttggaaggac catagatgag 300
aaaagaagcc ccgcagcacg ggtcaacatg gaggcaggga ccaggagcca tttccccagc 360
ctgccccaaa ggtttgggag aacaacagcc agaagcccca agacacccgc tgatttgcca 420
cagaaacccc tgcactcact gggctccagc gagttgctct acgtcatgat ctgccagcac 480
caagaaattc agagtcctgg tggaaagcga acgaggagag gagcgtttgt ggaaacagat 540
gatgcagaaa ggaaaccaga aaaa 564

<210> 27
<211> 203
<212> PRT
<213> Rat

<400> 27
Met Glu Ile Ile Ser Ser Lys Arg Phe Ile Leu Leu Thr Leu Ala Thr
1               5                   10                  15
Ser Ser Phe Leu Thr Ser Asn Thr Leu Cys Ser Asp Glu Leu Met Met
            20                  25                  30
Pro His Phe His Ser Lys Glu Gly Tyr Gly Lys Tyr Tyr Gln Leu Arg
        35                  40                  45
Gly Ile Pro Lys Gly Val Lys Glu Arg Ser Val Thr Phe Gln Glu Leu
    50                  55                  60
Lys Asp Trp Gly Ala Lys Lys Asp Ile Lys Met Ser Pro Ala Pro Ala
65                  70                  75                  80
Asn Lys Val Pro His Ser Ala Ala Asn Leu Pro Leu Arg Phe Gly Arg
                85                  90                  95
Asn Ile Glu Asp Arg Arg Ser Pro Arg Ala Arg Ala Asn Met Glu Ala
            100                 105                 110
Gly Thr Met Ser His Phe Pro Ser Leu Pro Gln Arg Phe Gly Arg Thr
        115                 120                 125

Thr Ala Arg Arg Ile Thr Lys Thr Leu Ala Gly Leu Pro Gln Lys Ser
    130                 135                 140
Leu His Ser Leu Ala Ser Ser Glu Leu Leu Tyr Ala Met Thr Arg Gln
145                 150                 155                 160
His Gln Glu Ile Gln Ser Pro Gly Gln Glu Gln Pro Arg Lys Arg Val
                165                 170                 175
Phe Thr Glu Thr Asp Asp Ala Glu Arg Lys Gln Glu Lys Ile Gly Asn
            180                 185                 190
Leu Gln Pro Val Leu Gln Gly Ala Met Lys Leu
        195                 200

<210> 28
<211> 609
<212> DNA
<213> Rat

<400> 28
atggaaatta tttcatcaaa gcgattcatt ttattgactt tagcaacttc aagcttctta 60
acttcaaaca ccctttgttc agatgaatta atgatgcccc attttcacag caaagaaggt 120
tatggaaaat attaccagct gagaggaatc ccaaaagggg taaaggaaag aagtgtcact 180
tttcaagaac tcaaagattg gggggcaaag aaagatatta agatgagtcc agcccctgcc 240
aacaaagtgc cccactcagc agccaacctt cccctgaggt ttgggaggaa catagaagac 300
agaagaagcc ccagggcacg ggccaacatg gaggcaggga ccatgagcca ttttcccagc 360
ctgccccaaa ggtttgggag aacaacagcc agacgcatca ccaagacact ggctggtttg 420
ccccagaaat ccctgcactc cctggcctcc agtgaattgc tctatgccat gacccgccag 480
catcaagaaa ttcagagtcc tggtcaagag caacctagga aacgggtgtt cacggaaaca 540
gatgatgcag aaaggaaaca agaaaaaata ggaaacctcc agccagtcct tcaaggggct 600
atgaagctg 609

<210> 29
<211> 203
<212> PRT
<213> Rat

<400> 29
Met Glu Ile Ile Ser Ser Lys Arg Phe Ile Leu Leu Thr Leu Ala Thr

1               5                   10                  15
Ser Ser Phe Leu Thr Ser Asn Thr Leu Cys Ser Asp Glu Leu Met Met
            20                  25                  30
Pro His Phe His Ser Lys Glu Gly Tyr Gly Lys Tyr Tyr Gln Leu Arg
        35                  40                  45
Gly Ile Pro Lys Gly Val Lys Glu Arg Ser Val Thr Phe Gln Glu Leu
    50                  55                  60
Lys Asp Trp Gly Ala Lys Lys Asp Ile Lys Met Ser Pro Ala Pro Ala
65                  70                  75                  80
Asn Lys Val Pro His Ser Ala Ala Asn Leu Pro Leu Arg Phe Gly Arg
                85                  90                  95
Asn Ile Glu Asp Arg Arg Ser Pro Arg Ala Arg Ala Asn Met Glu Ala
            100                 105                 110
Gly Thr Met Ser His Phe Pro Ser Leu Pro Gln Arg Phe Gly Arg Thr
        115                 120                 125
Thr Ala Arg Arg Ile Thr Lys Thr Leu Ala Gly Leu Pro Gln Lys Ser
    130                 135                 140
Leu His Ser Leu Ala Ser Ser Glu Ser Leu Tyr Ala Met Thr Arg Gln
145                 150                 155                 160
His Gln Glu Ile Gln Ser Pro Gly Gln Glu Gln Pro Arg Lys Arg Val
                165                 170                 175
Phe Thr Glu Thr Asp Asp Ala Glu Arg Lys Gln Glu Lys Ile Gly Asn
            180                 185                 190
Leu Gln Pro Val Leu Gln Gly Ala Met Lys Leu
        195                 200

<210> 30
<211> 609
<212> DNA
<213> Rat

<400> 30
atggaaatta tttcatcaaa gcgattcatt ttattgactt tagcaacttc aagcttctta    60
acttcaaaca ccctttgttc agatgaatta atgatgcccc attttcacag caaagaaggt   120
tatggaaaat attaccagct gagaggaatc ccaaaagggg taaaggaaag aagtgtcact   180
tttcaagaac tcaaagattg gggggcaaag aaagatatta agatgagtcc agcccctgcc   240

aacaaagtgc cccactcagc agccaacctt cccctgaggt ttgggaggaa catagaagac 300
agaagaagcc ccagggcacg ggccaacatg gaggcaggga ccatgagcca ttttcccagc 360
ctgccccaaa ggtttgggag aacaacagcc agacgcatca ccaagacact ggctggtttg 420
ccccagaaat ccctgcactc cctggcctcc agtgaatcgc tctatgccat gacccgccag 480
catcaagaaa ttcagagtcc tggtcaagag caacctagga aacgggtgtt cacggaaaca 540
gatgatgcag aaaggaaaca agaaaaaata ggaaacctcc agccagtcct tcaaggggct 600
atgaagctg 609

## Description

### TECHNICAL FIELD

The present invention relates to a method of producing a partial peptide of a polypeptide comprising the amino acid sequence represented by SEQ ID NO: 19, NO: 21, NO: 23, NO: 25, NO: 27 or NO: 29, or a salt thereof, which comprises producing a fusion protein or polypeptide, and then subjecting said fusion protein or polypeptide to a reaction of cleaving a peptide bond. Further, the present invention relates to a method of efficiently eliminating the N-terminal Met residue, optionally oxidized, or a diketone of the Met residue from a partial peptide of a polypeptide comprising the amino acid sequence represented by SEQ ID NO: 19, NO: 21, NO: 23, NO: 25, NO: 27 or NO: 29 having a Met residue at the N-terminal, optionally oxidized, or a salt thereof, if desired.

### BACKGROUND ART

In the production of a peptide by recombinant DNA technology, it is more or less common practice to express the peptide in the form of a fusion protein because of frequent decomposition of the peptide within cells. For excision of the target peptide from the fusion protein, a chemical cleavage using cyanogen bromide (Itakura et al., Science, 198, 1056, 1977) and an enzymatic cleavage using factor Xa (Nagai et al., Methods in Enzymology, 153, 46, 1987) are known.

Further, as a method for cleavage of a peptide bond in a protein, cleavage of the acylcysteine bond with 2-nitro-5-thiocyanobenzoic acid is known ("Seikagaku Jikken Koza 1, Tanpakushitsu-no-Kagaku II (Biochemical Experiment Series 1, Protein Chemistry II)", Japanese Society of Biochemistry ed., Tokyo Kagaku Dojin, 247-250, 1976). However, there is no disclosure on the excision of a partial peptide of a polypeptide comprising the amino acid sequence represented by SEQ ID NO: 19, NO: 21, NO: 23, NO: 25, NO: 27 or NO: 29 from a protein.

The prior art method using cyanogen bromide cannot be applied to the production of methionine-containing peptides, and has drawbacks, for example in terms of excision yield.

Therefore, there is a need for a method of efficiently excising a partial peptide of a polypeptide comprising the amino acid sequence represented by SEQ ID NO: 19, NO: 21, NO: 23, NO: 25, NO: 27 or NO: 29 from a fusion protein or polypeptide.

In addition, there may be differences in higher structure, biological activity, and stability between the molecular type containing Met at the N-terminal and the one not containing Met at the N-terminal of a partial peptide of a polypeptide comprising the amino acid sequence represented by SEQ ID NO: 19, NO: 21, NO: 23, NO: 25, NO: 27 or NO: 29. The addition of Met to the N-terminal may also enhance the antigenecity of the partial peptide. Therefore, in view of industrial applicability, it is significant to establish a method of eliminating the N-terminal Met as the initiation codon.

### SUMMARY OF THE INVENTION

As a result of intensive research on a method of efficiently producing a partial peptide of the polypeptide comprising the amino acid sequence of SEQ ID NO: 19, 21, 23, 25, 27 or 29, or a salt thereof, we discovered that the partial peptide of the polypeptide comprising the amino acid sequence of SEQ ID NO: 19, 21, 23, 25, 27 or 29, or a salt thereof can be efficiently produced by preparing a fusion protein or polypeptide, in which the partial peptide of the polypeptide comprising the amino acid sequence of SEQ ID NO: 19, 21, 23, 25, 27 or 29 is ligated to the N-terminal of a protein or polypeptide having a cysteine at the N-terminal, and then subjecting the fusion protein or polypeptide to the reaction for cleavage of a peptide bond. We did further research on the basis of the above finding and accomplished the present invention.

When produced according to the production method shown above, in particular, the polypeptide having the amino acid sequence shown in SEQ ID NO: 3, 5, 9 or 11 may possibly have an extra methionine residue added at the N-terminal. Thus, we made further intensive researched into a method of producing a partial peptide of the polypeptide comprising the amino acid sequence of SEQ ID NO: 19, 21, 23, 25, 27 or 29 in a form having the naturally-occurring amino acid sequence, which comprises excising only the N-terminal methionine residue from the partial peptide of the polypeptide comprising the amino acid sequence of SEQ ID NO: 19, 21, 23, 25, 27 or 29 produced by the genetic engineering. As a result, we found that, as shown in Scheme I, a transamination reaction is carried out by reacting a partial peptide of the polypeptide comprising the amino acid sequence of SEQ ID NO: 19, 21, 23, 25, 27 or 29, which has a methionine residue optionally oxidized at the N-terminal, as represented by Formula 1, with, for example, α-diketone such as glyoxylic acid, a donor offering transition metal ions such as copper sulfate, and a base (e.g. amines) such as pyridine; and a subsequent hydrolysis reaction is carried out by reacting the partial peptide of the polypeptide comprising the amino acid sequence of SEQ ID NO: 19, 21, 23, 25, 27 or 29, which has the thus obtained diketone of the methionine residue optionally oxidized at the N-terminal, with a base (e.g. diamines) such as 3,4-diamino-benzoic acid, so that the diketone of methionine residue can be removed unexpectedly efficiently from the partial peptide having the diketone. This means our finding of a method of producing unexpectedly efficiently a partial peptide of the polypeptide comprising the amino acid sequence of SEQ ID NO: 19, 21, 23, 25, 27 or 29, which does not have a methionine residue optionally oxidized at the N-terminal, which comprises removing the N-terminal methionine residue optionally oxidized from the partial peptide of the polypeptide comprising the amino acid sequence of SEQ ID NO: 19, 21, 23, 25, 27 or 29, which has the methionine residue, without a decrease in its activity. We finally accomplished the present invention after further research thereon.

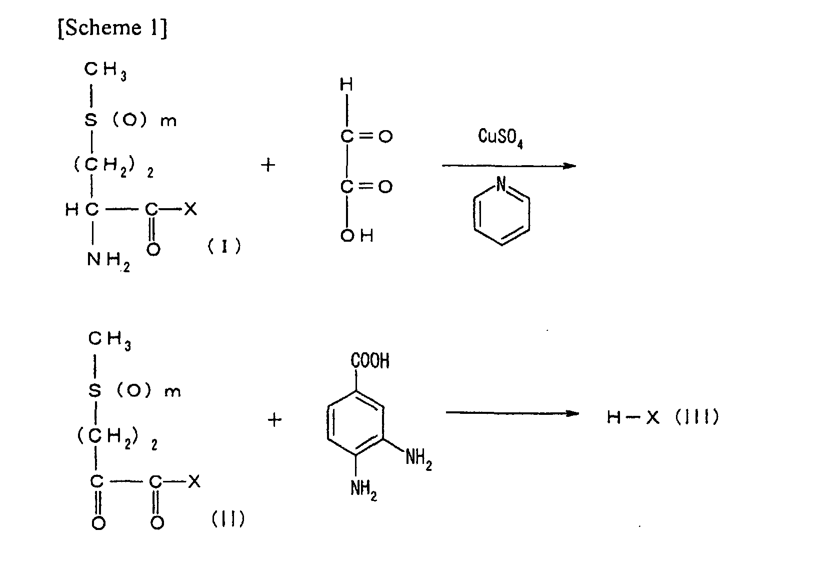

In Scheme I, m indicates an integer of 0 to 2, and X indicates a partial peptide chain of the polypeptide comprising the amino acid sequence represented by SEQ ID NO: 19, 21, 23, 25, 27 or 29.

In the present specification and Scheme I,
(1) The compound represented by the general formula (I) may refer to "a partial peptide of the polypeptide comprising the amino acid sequence of SEQ ID NO: 19, 21, 23, 25, 27 or 29, which has a methionine residue optionally oxidized at the N-terminal, or a salt thereof" or "a partial peptide of the polypeptide comprising the amino acid sequence of SEQ ID NO: 19, 21, 23, 25, 27 or 29, which has a methionine residue, or a salt thereof';
(2) In the general formula (I): 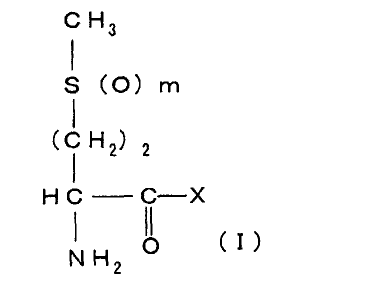 wherein m is defined as above,
   the moiety shown above may refer to "a methionine residue optionally oxidized", "a methionine residue" or "a methionine";
(3) The compound represented by the general formula (II) may refer to "a partial peptide of the polypeptide comprising the amino acid sequence of SEQ ID NO: 19, 21, 23, 25, 27 or 29, which has a diketone of methionine residue optionally oxidized at the N-terminal, or a salt thereof" or "the polypeptide comprising the amino acid sequence of SEQ ID NO: 19, 21, 23, 25, 27 or 29, which has a diketone of methionine residue, or a salt thereof";
(4) In the general formula (II): 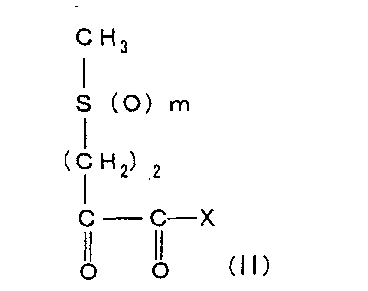 wherein m is defined as above,
   the moiety shown above may refer to "a diketone of methionine residue optionally oxidized" or "a diketone of methionine residue";
(5) The compound represented by the general formula (III) may refer to "the polypeptide comprising the amino acid sequence of SEQ ID NO: 19, 21, 23, 25, 27 or 29, which does not have a methionine residue optionally oxidized at the N-terminal, or a salt thereof" or "the polypeptide comprising the amino acid sequence of SEQ ID NO: 19, 21, 23, 25, 27 or 29, which does not have a diketone of methionine residue optionally oxidized at the N-terminal, or a salt thereof".

Thus, the present invention provides:
(1) A method of producing a partial peptide of the polypeptide comprising the amino acid sequence of SEQ ID NO: 19, 21, 23, 25, 27 or 29, or a salt thereof, which comprises:
   subjecting a fusion protein or polypeptide, in which a partial peptide of the polypeptide comprising the amino acid sequence of SEQ ID NO: 19, 21, 23, 25, 27 or 29, which may has a methionine residue optionally oxidized at the N-terminal, is ligated to the N-terminal of a protein or polypeptide having a cysteine at the N-terminal, to a reaction for cleavage of a peptide bond on the amino group side of the cysteine residue.
(2) A method of producing a partial peptide of the polypeptide comprising the amino acid sequence of SEQ ID NO: 19,21,23, 25, 27 or 29, which may has a methionine residue at the N-terminal, or a salt thereof, which comprises:
   culturing a transformant containing a vector having a DNA encoding a fusion protein or polypeptide, in which the partial peptide of the polypeptide comprising the amino acid sequence of SEQ ID NO: 19, 21, 23, 25, 27 or 29, which may has a methionine residue at the N-terminal, is ligated to the N-terminal of a protein or polypeptide having a cysteine at the N-terminal to express the fusion protein or polypeptide; and
   subjecting the expressed fusion protein or polypeptide to a reaction for cleavage of a peptide bond on the amino group side of the cysteine residue.
(3) The production method of (1) or (2), wherein the cleavage reaction is S-cyanation reaction followed by an ammonolysis or hydrolysis reaction.
(4) The production method of (1) or (2), wherein the partial peptide of the polypeptide comprising the amino acid sequence of SEQ ID NO: 19, 21, 23, 25, 27 or 29 includes:
   (i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
   (ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
   (iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
   (iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
   (v) a peptide comprising the amino acid sequence from aa 58 (Ser) to aa 94 (Phe), or aa 84 (Pro) to aa 94 (Phe) in the amino acid sequence of SEQ ID NO: 27.
(5) The production method of (1) or (2), wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.
(6) The production method of (5), wherein the polypeptide comprising the amino acid sequence of SEQ ID NO: 1 or 9 includes the polypeptide having the amino acid sequence of SEQ ID NO: 1, 3, 5, 7, 9 or 11.
(7) A fusion protein or polypeptide, in which a partial peptide of the polypeptide comprising the amino acid sequence of SEQ ID NO: 19, 21, 23, 25, 27 or 29, which may has a methionine residue at the N-terminal, is ligated to the N-terminal of a protein or polypeptide having a cysteine at the N-terminal, or a salt thereof.
(8) The polypeptide of (7) or a salt thereof, wherein the partial peptide of the polypeptide comprising the amino acid sequence of SEQ ID NO: 19, 21, 23, 25, 27 or 29 includes:
   (i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa I (Met) to aa 92 (Phe), aa 1 (Met) to aa 112 (Ser), or aa 1 (Met) to aa 131 (Phe) in the amino acid sequence of SEQ ID NO: 19;
   (ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
   (iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
   (iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
   (v) a peptide comprising the amino acid sequence from aa 58 (Ser) to aa 94 (Phe), or aa 84 (Pro) to aa 94 (Phe) in the amino acid sequence of SEQ ID NO: 27.
(9) The polypeptide of (7) or a salt thereof, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.
(10) The polypeptide of (9) or a salt thereof, wherein the polypeptide comprising the amino acid sequence of SEQ ID NO: 1 or 9 includes the polypeptide having the amino acid sequence of SEQ ID NO: 1, 3, 5, 7, 9 or 11.
(11) A vector comprising the DNA encoding the fusion protein or polypeptide of (7).
(12) A transformant having the vector of (11).
(13) A method of removing the N-terminal methionine residue from a partial peptide of the polypeptide comprising the amino acid sequence of SEQ ID NO: 19, 21, 23, 25, 27 or 29, which has a methionine residue optionally oxidized at the N-terminal, which comprises:
   reacting the partial peptide or a salt thereof with α-diketone; and then
   subjecting it to a hydrolysis reaction.
(14) The method of (13), wherein the partial peptide of the polypeptide comprising the amino acid sequence of SEQ ID NO: 19, 21, 23, 25, 27 or 29, which has a methionine residue optionally oxidized at the N-terminal, is produced by genetic engineering, and the amino acid next to the methionine residue on the C-terminal side is Isoleucine, Valine, Cysteine, Threonine, Aspartic acid, Lysine, Leucine, Arginine, Asparagine, Methionine, Phenylalanine, Tyrosine, Tryptophan, Glutamic acid, Glutamine or Histidine.
(15) The method of (13) or (14), wherein the partial peptide of the polypeptide comprising the amino acid sequence of SEQ ID NO: 19, 21, 23, 25, 27 or 29 includes:
   (i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
   (ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa I (Met) to aa 131 (Phe) in the amino acid sequence of SEQ ID NO: 21;
   (iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
   (iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
   (v) a peptide comprising the amino acid sequence from aa 58 (Ser) to aa 94 (Phe), or aa 84 (Pro) to aa 94 (Phe) in the amino acid sequence of SEQ ID NO: 27.
(16) The method of (13) or (14), wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.
(17) The method of (16), wherein the polypeptide comprising the amino acid sequence of SEQ ID NO: 1 or 9 includes the polypeptide having the amino acid sequence of SEQ ID NO: 1,3, 5, 7, 9 or 11.
(18) The method of (13), wherein the reaction with a-diketone is carried out in the presence of a transition metal ion.
(19) The method of (13), wherein the reaction with α-diketone is carried out in the presence of a base.
(20) The method of (13), wherein the reaction with α-diketone is carried out in the presence of a transition metal ion and a base.
(21) The method of (13), wherein the α-diketone is glyoxylic acid or its salt.
(22) The method of (18), wherein the transition metal ion is a copper ion.
(23) The method of (19), wherein the base is pyridine.
(24) The method of (13), wherein the hydrolysis reaction is carried out using a base.
(25) The method of (24), wherein the base is an amine.
(26) The method of (24), wherein the base is a diamine, or thio- or selenosemicarbazide.
(27) The method of (26), wherein the diamine is o-phenylenediamine or 3,4-diamino-benzoic acid.
(28) A method of producing the polypeptide comprising the amino acid sequence of SEQ ID NO: 1 or 9, or a salt thereof, which comprises:
   reacting a partial peptide of the polypeptide comprising the amino acid sequence of SEQ ID NO: 19, 21, 23, 25, 27 or 29, which has a methionine residue added at the N-terminal, or a salt thereof, which is produced by genetic engineering, with glyoxylic acid or its salt in the presence of copper sulfate and pyridine; and then
   reacting it with o-phenylenediamine or 3,4-diamino-benzoic acid.
(29) The production method of (28), wherein the partial peptide of the polypeptide comprising the amino acid sequence of SEQ ID NO: 19, 21, 23, 25, 27 or 29 includes:
   (i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa I (Met) to aa 92 (Phe), aa 1 (Met) to aa 112 (Ser), or aa 1 (Met) to aa 131 (Phe) in the amino acid sequence of SEQ ID NO: 19;
   (ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
   (iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
   (iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
   (v) a peptide comprising the amino acid sequence from aa 58 (Ser) to aa 94 (Phe), or aa 84 (Pro) to aa 94 (Phe) in the amino acid sequence of SEQ ID NO: 27.
(30) The production method of (28), wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.
(31) The production method of (30), wherein the polypeptide comprising the amino acid sequence of SEQ ID NO: 1 or 9 includes the polypeptide having the amino acid sequence of SEQ ID NO: 1, 3, 5, 7, 9 or 11.
(32) A compound represented by the formula CH₃-S(O)ₘ-(CH₂)₂-CO-CO-X, or a salt thereof, wherein m indicates an integer of 0 to 2, and X indicates a partial peptide chain of the polypeptide comprising the amino acid sequence of SEQ ID NO: 19, 21, 23, 25,27 or 29.
(33) The compound of (32), or a salt thereof, wherein the partial peptide of the polypeptide comprising the amino acid sequence of SEQ ID NO: 19, 21, 23, 25, 27 or 29 includes:
   (i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
   (ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
   (iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
   (iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
   (v) a peptide comprising the amino acid sequence from aa 58 (Ser) to aa 94 (Phe), or aa 84 (Pro) to aa 94 (Phe) in the amino acid sequence of SEQ ID NO: 27.
(34) The compound of (32), or a salt thereof, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.
(35) The compound of (34), or a salt thereof, wherein the polypeptide comprising the amino acid sequence of SEQ ID NO: 1 or 9 includes the polypeptide having the amino acid sequence of SEQ ID NO: 1, 3, 5, 7, 9 or 1.
(36) A method of producing a partial peptide of the polypeptide comprising the amino acid sequence of SEQ ID NO: 19, 21, 23, 25, 27 or 29, or a salt thereof, which comprises subjecting the compound of (32) to a hydrolysis reaction.
(37) A method of producing the polypeptide comprising the amino acid sequence represented by, or a salt thereof, which comprises:
   subjecting a fusion protein or polypeptide, in which the polypeptide comprising the amino acid sequence represented by , which has a methionine residue at the N-terminal, is ligated to the N-terminal of a protein or polypeptide having a cysteine at the N-terminal, to a reaction for cleavage of a peptide bond on the amino group side of the cysteine residue to obtain the polypeptide comprising the amino acid sequence represented by , which has a methionine residue optionally oxidized at the N-terminal, or a salt thereof;
   reacting the polypeptide comprising the amino acid sequence represented by , which has a methionine residue optionally oxidized at the N-terminal, or a salt thereof with α-diketone; and
   subjecting it to a hydrolysis reaction.
(38) The production method of (37), wherein the partial peptide of the polypeptide comprising the amino acid sequence of SEQ ID NO: 19, 21, 23, 25, 27 or 29 includes:
   (i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
   (ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or as 1 (Met) to aa 131 (Phe) in the amino acid sequence of SEQ ID NO: 21;
   (iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
   (iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
   (v) a peptide comprising the amino acid sequence from aa 58 (Ser) to aa 94 (Phe), or aa 84 (Pro) to aa 94 (Phe) in the amino acid sequence of SEQ ID NO: 27.
(39) The production method of (37), wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.
(40) The production method of (39), wherein the polypeptide comprising the amino acid sequence of SEQ ID NO: 1 or 9 includes the polypeptide having the amino acid sequence of SEQ ID NO: 1, 3, 5, 7, 9 or 11.

### BRIEF DESCRIPTION OF THE DRAWINGS

Figure 1 indicates the reaction mechanism in the production method of the invention.
Figure 2 indicates a schematic diagram of the construction of the plasmid pTFCRFRP-1 obtained in Example 2.

### BEST MODE FOR CARRYING OUT THE INVENTION

The polypeptides comprising the amino acid sequence represented by SEQ ID NO: 19, NO: 21, NO: 23, NO: 25, NO: 27 or NO: 29 in the invention (sometimes referred to as the precursor polypeptide of the invention) are polypeptides comprising the amino acid sequence represented by SEQ ID NO: 1, NO: 8, NO: 14, NO: 33, NO: 50 or NO: 19, respectively, described in WO 00/29441 and WO 01/66134.

The precursor polypeptides of the invention are shown according to the conventional notation so that the N-terminal (amino terminal) is placed on the left side and the C-terminal (carboxyl terminal) on the right side. In the precursor polypeptides of the invention, including the precursor polypeptide comprising the amino acid sequence shown by SEQ ID NO: 1, their C-terminals are normally carboxyl group (-COOH) or carboxylate (-COO⁻), and may also be amide (-CONH₂) or ester (-COOR).

The ester group "R" includes C₁₋₆ alkyl group such as methyl, ethyl, n-propyl, isopropyl, or n-butyl; C₃₋₈ cycloalkyl group such as cyclopenthyl, or cyclohexyl; C₆₋₁₂ aryl group such as phenyl, or α-naphthyl; C₇₋₁₄ aralkyl group such as phenyl-C₁₋₂ alkyl, e.g. benzyl or phenethyl, or α-naphthyl-C₁₋₂ alkyl, e.g. α-naphthylmethyl; or pivaloyloxymethyl group generally used as an oral ester.

When the precursor polypeptide of the invention has a carboxyl group or carboxylate at a position other than the C-terminal, it may be amidated or esterified. Such an amide or ester form is also included in the polypeptide of the invention, The ester group may be the same as described with respect to the above C-terminal ester group.

Furthermore, the precursor polypeptide of the invention includes variants thereof, wherein the amino group at the N-terminal (e.g., methionine residue) is protected with a protecting group (e.g., C₁₋₆ acyl group such as C₁₋₆ alkanoyl, e.g. formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., C₁₋₆ acyl group such as C₁₋₆ alkanoyl group, e.g., fonnyl group, acetyl group, etc.), or conjugated proteins such as a glycoprotein having sugar chains. Hereinafter, the term "the precursor polypeptide of the invention" may be used to include these polypeptides.

The partial peptide of the precursor polypeptide of the invention (hereinafter occasionally referred to as the polypeptides of the invention) may be any partial peptides of the precursor polypeptide of the invention described above, for example, any one which can bind to a receptor protein of the precursor polypeptide (specifically, the protein comprising the same or substantially the same amino acid sequence as that shown in SEQ ID NO: 37 in WO 00/29441 and WO 01/66134, or a salt thereof) and has an activity of regulating prolactin secretion.

In the polypeptide of the invention, 1 to 5 (preferably 1 to 3) amino acids in the amino acid sequence ofthe polypeptide may be deleted; or 1 to 5 (preferably 1 to 3) amino acids may be added to the amino acid sequence; or 1 to 5 (preferably 1 to 3) amino acids may be inserted into the amino acid sequence; or 1 to 5 (preferably 1 to 3) amino acids in the amino acid sequence may be substituted by other amino acids; or a combination thereof may be included in the amino acid sequence.

In the polypeptides of the invention, the C-terminals are normally carboxyl group (-COOH) or carboxylate (-COO⁻), and may also be amide (-CONH₂) or ester (-COOR), as described in the precursor polypeptide of the invention (R is defined as described above). Particularly preferred is the polypeptide having amidated C-terminal (-CONH₂).

When the polypeptide of the invention has a carboxyl group (or carboxylate) at a position other than the C-terminal, it may be amidated or esterified. Such an amide or ester form is also included in the polypeptide of the invention. The ester group may be the same as described with respect to the above C-terminal ester group.

Furthermore, as well as the precursor polypeptide of the invention as described above, the polypeptide of the invention includes variants thereof, wherein the amino group at the N-terminal (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated proteins such as a glycoprotein having sugar chains. Hereinafter, the term "the polypeptide of the invention" may be used to include these polypeptides.

The partial peptide of the precursor polypeptide of the invention may be, for example, a peptide having RFamide, RS amide or RLamide structure, more preferably, a peptide having RFamide or RS amide structure, and particularly preferably, a peptide having RFamide structure.

The RFamide structure means that the C-terminal of the peptide is Arg-Phe-NH₂. The RSamide structure means that the C-terminal of the peptide is Arg-Ser-NH₂. The RLamide structure means that the C-terminal of the peptide is Arg-Leu-NH₂.

Preferred examples of the polypeptide of the invention include:
(1) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
(2) a peptide comprising the amino acid sequence from aa 56 (Ser) to aa 92 (Phe), aa 73 (Met) to aa 92 (Phe), aa 81 (Met) to aa 92 (Phe)_{,} aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
(3) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa I (Met) to aa 131 (Phe) in the amino acid sequence of SEQ ID NO: 23;
(4) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25;
(5) a peptide comprising the amino acid sequence from aa 58 (Ser) to aa 94 (Phe) or aa 84 (Pro) to aa 94 (Phe) in the amino acid sequence of SEQ ID NO: 27.

In particular, preferred examples include:
(1) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), or aa 124 (Val) to aa 131 (Phe) in the amino acid sequence of SEQ ID NO: 19;
(2) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), or aa 124 (Val) to aa 131 (Phe) in the amino acid sequence of SEQ ID NO: 21;
(3) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), or aa 124 (Val) to aa 131 (Phe) in the amino acid sequence of SEQ ID NO: 23;
(4) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25;
(5) a peptide comprising the amino acid sequence from aa 58 (Ser) to aa 94 (Phe) or aa 84 (Pro) to aa 94 (Phe) in the amino acid sequence of SEQ ID NO: 27.

In particular, preferred examples include:
(1) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), or aa 124 (Val) to aa 131 (Phe) in the amino acid sequence of SEQ ID NO: 19;
(2) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), or aa 124 (Val) to aa 131 (Phe) in the amino acid sequence of SEQ ID NO: 21;
(3) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), or aa 124 (Val) to aa 131 (Phe) in the amino acid sequence of SEQ ID NO: 23;
(4) a peptide comprising the amino acid sequence from aa 58 (Ser) to aa 94 (Phe), aa 83 (Val) to aa 94 (Phe) or aa 118 (Phe) to aa 125 (Phe) in the amino acid sequence of SEQ ID NO: 25;
(5) a peptide comprising the amino acid sequence from aa 58 (Ser) to aa 94 (Phe) in the amino acid sequence of SEQ ID NO: 27.

In particular, preferred examples include:
(1) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 115 (Asn) to aa 131 (Phe), or aa 124 (Val) to aa 131 (Phe) in the amino acid sequence of SEQ ID NO: 19;
(2) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 115 (Asn) to aa 131 (Phe), or aa 124 (Val) to aa 131 (Phe) in the amino acid sequence of SEQ ID NO: 21;
(3) a peptide comprising the amino acid sequence from aa 58 (Ser) to aa 92 (Phe), aa 81 (Met) to aa 92 (Phe) or aa 124 (Val) to aa 131 (Phe) in the amino acid sequence of SEQ ID NO: 23.

In particular, preferred examples include:
(1) a peptide comprising the amino acid sequence from aa 56 (Ser) to aa 92 (Phe), aa 73 (Met) to aa 92 (Phe), aa 81 (Met) to aa 92(Phe), or aa 84 (Ser) to aa 92 (Phe) in the amino acid sequence of SEQ ID NO: 19;
(2) a peptide comprising the amino acid sequence from aa 56 (Ser) to aa 92 (Phe), aa 73 (Met) to aa 92 (Phe), aa 81 (Met) to aa 92 (Phe), or aa 84 (Ser) to aa 92 (Phe) in the amino acid sequence of SEQ ID NO: 21;
(3) a peptide comprising the amino acid sequence from aa 58 (Ser) to aa 92 (Phe) or aa 81 (Met) to aa 92 (Phe) in the amino acid sequence of SEQ ID NO: 23.

In particular, preferred examples include:
(1) a peptide comprising the amino acid sequence from aa 81 (Met) to aa 92(Phe) in the amino acid sequence of SEQ ID NO: 19;
(2) a peptide comprising the amino acid sequence from aa 81 (Met) to aa 92 (Phe) in the amino acid sequence of SEQ ID NO: 21;
(3) a peptide comprising the amino acid sequence from aa 81 (Met) to aa 92 (Phe) in the amino acid sequence of SEQ ID NO: 23.

In particular, preferred examples include:
(1) a peptide comprising the amino acid sequence from aa 81 (Met) to aa 92(Phe) in the amino acid sequence of SEQ ID NO: 19;
(2) a peptide comprising the amino acid sequence from aa 81 (Met) to aa 92 (Phe) in the amino acid sequence of SEQ ID NO: 21.

Further, preferred examples of the polypeptide of the invention include:
(a) a peptide having the amino acid sequence from aa 81 (Met) to aa 92 (Phe) in the amino acid sequence of SEQ ID NO: 19;
(b) a peptide having the amino acid sequence from aa 101 (Ser) to aa 112 (Ser) in the amino acid sequence of SEQ ID NO: 19;
(c) a peptide having the amino acid sequence from aa 124 (Val) to aa 131 (Phe) in the amino acid sequence of SEQ ID NO: 19;
(d) a peptide having the amino acid sequence from aa 56 (Ser) to aa 92 (Phe) in the amino acid sequence of SEQ ID NO: 19;
(e) a peptide having the amino acid sequence from aa 81 (Met) to aa 92 (Phe) in the amino acid sequence of SEQ ID NO: 23;
(f) a peptide having the amino acid sequence from aa 101 (Ser) to aa 112 (Leu) in the amino acid sequence of SEQ ID NO: 23;
(g) a peptide having the amino acid sequence from aa 58 (Ser) to aa 92 (Phe) in the amino acid sequence of SEQ ID NO: 23;
(h) a peptide having the amino acid sequence from aa 83 (Val) to aa 94 (Phe) in the amino acid sequence of SEQ ID NO: 25;
(i) a peptide having the amino acid sequence from aa 118 (Phe) to aa 125 (Phe) in the amino acid sequence of SEQ ID NO: 25;
(j) a peptide having the amino acid sequence from aa 58 (Ser) to aa 94 (Phe) in the amino acid sequence of SEQ ID NO: 25;
(k) a peptide having the amino acid sequence from aa 58 (Ser) to aa 94 (Phe) in the amino acid sequence of SEQ ID NO: 27.

In particular, these peptides are preferably in the amide form (wherein, preferably, the C-terminal carboxyl group (-COOH) is amidated (-CONH₂)).

Examples of these peptides in the amide form include:
a peptide having the amino acid sequence from aa 81 (Met) to aa 92 (Phe) in the amino acid sequence of SEQ ID NO: 19, wherein the C-terminal is amidated (-CONH₂);
a peptide having the amino acid sequence from aa 101 (Ser) to aa 112 (Ser) in the amino acid sequence of SEQ ID NO: 19, wherein the C-terminal is amidated (-CONH₂);
a peptide having the amino acid sequence from aa 124 (Val) to aa 131 (Phe) in the amino acid sequence of SEQ ID NO: 19, wherein the C-terminal is amidated (-CONH₂).

Among them, preferred are a peptide having the amino acid sequence from aa 81 (Met) to aa 92 (Phe) in the amino acid sequence of SEQ ID NO: 19, wherein the C-terminal is amidated (-CONH₂); and a peptide having the amino acid sequence from aa 124 (Val) to aa 131 (Phe) in the amino acid sequence of SEQ ID NO: 19, wherein the C-terminal is amidated (-CONH₂). In particular, preferred is a peptide having the amino acid sequence from aa 81 (Met) to aa 92 (Phe) in the amino acid sequence of SEQ ID NO: 19, wherein the C-terminal is amidated (-CONH₂).

In addition, as the polypeptide of the invention, for example, a polypeptide comprising the amino acid sequence shown in SEQ ID NO: 1 or 9 may preferably be used. Specifically, any polypeptides comprising the amino acid sequence shown in SEQ ID NO: 1 or 9, for example, RFamide-related peptides described in Hinuma et al., Nature Cell Biology, Vol.2, p.703-708 (2000) and polypeptides described in WO 00/29441, and having an ligand activity to the receptor 0T7T022 described in Hinuma et al., Nature Cell Biology, Vol.2, p.703-708 (2000) and WO 00/29441 may be used.

Such polypeptides include the polypeptide having the amino acid sequence represented by SEQ ID NO: 1, 3, 5, 7, 9 or 11.

Examples of the DNA encoding the precursor polypeptide of the invention include:
(1) For the DNA encoding the human precursor polypeptide comprising the amino acid sequence of SEQ ID NO: 19, a DNA comprising the nucleotide sequence of SEQ ID NO: 20;
(2) For the DNA encoding the human precursor polypeptide comprising the amino acid sequence of SEQ ID NO: 21, a DNA comprising the nucleotide sequence of SEQ ID NO: 22;
(3) For the DNA encoding the bovine precursor polypeptide comprising the amino acid sequence of SEQ ID NO: 23, a DNA comprising the nucleotide sequence of SEQ ID NO: 24;
(4) For the DNA encoding the mouse precursor polypeptide comprising the amino acid sequence of SEQ ID NO: 25, a DNA comprising the nucleotide sequence of SEQ ID NO: 26;
(5) For the DNA encoding the rat precursor polypeptide comprising the amino acid sequence of SEQ ID NO: 27, a DNA comprising the nucleotide sequence of SEQ ID NO: 28;
(6) For the DNA encoding the rat precursor polypeptide comprising the amino acid sequence of SEQ ID NO: 29, a DNA comprising the nucleotide sequence of SEQ ID NO: 30.

The DNA encoding the polypeptide of the invention may be a DNA having a partial nucleotide sequence of the DNA comprising the nuleoitde sequence represented by SEQ ID NO: 20, 22, 24, 26, 28 or 30.

Examples of the DNA encoding the partial peptide of the invention include:
(1) For the DNA encoding a peptide comprising the sequence from aa 81 (Met) to aa 92(Phe) in the amino acid sequence of SEQ ID NO: 19, the DNA comprising the sequence from base 241 to base 276 in the base sequence of SEQ ID NO: 20;
(2) For the DNA encoding a peptide comprising the sequence from aa 101 (Ser) to aa 112 (Ser) in the amino acid sequence of SEQ ID NO: 19, the DNA comprising the sequence from base 301 to base 336 in the base sequence of SEQ ID NO: 20;
(3) For the DNA encoding a peptide comprising the sequence from aa 124 (Val) to aa 131 (Phe) in the amino acid sequence of SEQ ID NO: 19, the DNA comprising the sequence from base 370 to base 393 in the base sequence of SEQ ID NO: 20;
(4) For the DNA encoding a peptide comprising the sequence from aa 1 (Met) to aa 92 (Phe) in the amino acid sequence of SEQ ID NO: 19, the DNA comprising the sequence from base 1 to base 276 in the base sequence of SEQ ID NO: 20;
(5) For the DNA encoding a peptide comprising the sequence from aa 1 (Met) to aa 112 (Ser) in the amino acid sequence of SEQ ID NO: 19, the DNA comprising the sequence from base 1 to base 336 in the base sequence of SEQ ID NO: 20;
(6) For the DNA encoding a peptide comprising the sequence from aa 1 (Met) to aa 131 (Phe) in the amino acid sequence of SEQ ID NO: 19, the DNA comprising the sequence from base 1 to base 393 in the base sequence of SEQ ID NO: 20;
(7) For the DNA encoding a peptide comprising the sequence from aa 56 (Ser) to aa 92 (Phe) in the amino acid sequence of SEQ ID NO: 19, the DNA comprising the sequence from base 166 to base 276 in the base sequence of SEQ ID NO: 20;
(8) For the DNA encoding a peptide comprising the sequence from aa 73 (Met) to aa 92 (Phe) in the amino acid sequence of SEQ ID NO: 19, the DNA comprising the sequence from base 217 to base 276 in the base sequence of SEQ ID NO: 20;
(9) For the DNA encoding a peptide comprising the sequence from aa 84 (Ser) to aa 92 (Phe) in the amino acid sequence of SEQ ID NO: 19, the DNA comprising the sequence from base 253 to base 276 in the base sequence of SEQ ID NO: 20;
(10) For the DNA encoding a peptide comprising the sequence from aa 95 (Asn) to aa 112 (Ser) in the amino acid sequence of SEQ ID NO: 19, the DNA comprising the sequence from base 283 to base 393 in the base sequence of SEQ ID NO: 20;
(11) For the DNA encoding a peptide comprising the sequence from aa 115 (Asn) to aa 131 (Phe) in the amino acid sequence of SEQ ID NO: 19, the DNA comprising the sequence from base 346 to base 393 in the base sequence of SEQ ID NO: 20;
(12) For the DNA encoding a peptide comprising the sequence from aa 56 (Ser) to aa 92 (Phe) in the amino acid sequence of SEQ ID NO: 21, the DNA comprising the sequence from base 169 to base 276 in the base sequence of SEQ ID NO: 22;
(13) For the DNA encoding a peptide comprising the sequence from aa 73 (Met) to aa 92 (Phe) in the amino acid sequence of SEQ ID NO: 21, the DNA comprising the sequence from base 220 to base 276 in the base sequence of SEQ ID NO: 22;
(14) For the DNA encoding a peptide comprising the sequence from aa 81 (Met) to aa 92 (Phe) in the amino acid sequence of SEQ ID NO: 21, the DNA comprising the sequence from base 244 to base 276 in the base sequence of SEQ ID NO: 22;
(15) For the DNA encoding a peptide comprising the sequence from aa 84 (Ser) to aa 92 (Phe) in the amino acid sequence of SEQ ID NO: 21, the DNA comprising the sequence from base 253 to base 276 in the base sequence of SEQ ID NO: 22;
(16) For the DNA encoding a peptide comprising the sequence from aa 101 (Ser) to aa 112 (Ser) in the amino acid sequence of SEQ ID NO: 21, the DNA comprising the sequence from base 304 to base 336 in the base sequence of SEQ ID NO: 22;
(17) For the DNA encoding a peptide comprising the sequence from aa 95 (Asn) to aa 112 (Ser) in the amino acid sequence of SEQ ID NO: 21, the DNA comprising the sequence from base 283 to base 393 in the base sequence of SEQ ID NO: 22;
(18) For the DNA encoding a peptide comprising the sequence from aa 115 (Asn) to aa 131 (Phe) in the amino acid sequence of SEQ ID NO: 21, the DNA comprising the sequence from base 346 to base 393 in the base sequence of SEQ ID NO: 22;
(19) For the DNA encoding a peptide comprising the sequence from aa 124 (Val) to aa 131 (Phe) in the amino acid sequence of SEQ ID NO: 21, the DNA comprising the sequence from base 373 to base 393 in the base sequence of SEQ ID NO: 22;
(20) For the DNA encoding a peptide comprising the sequence from aa 1 (Met) to aa 92 (Phe) in the amino acid sequence of SEQ ID NO: 21, the DNA comprising the sequence from base 1 to base 276 in the base sequence of SEQ ID NO: 22;
(21) For the DNA encoding a peptide comprising the sequence from aa 1 (Met) to aa 112 (Ser) in the amino acid sequence of SEQ ID NO: 21, the DNA comprising the sequence from base 1 to base 336 in the base sequence of SEQ ID NO: 22;
(22) For the DNA encoding a peptide comprising the sequence from aa 1 (Met) to aa 131 (Phe) in the amino acid sequence of SEQ ID NO: 21, the DNA comprising the sequence from base 1 to base 393 in the base sequence of SEQ ID NO: 22;
(23) For the DNA encoding a peptide comprising the sequence from aa 58 (Ser) to aa 92 (Phe) in the amino acid sequence of SEQ ID NO: 23, the DNA comprising the sequence from base 172 to base 276 in the base sequence of SEQ ID NO: 24;
(24) For the DNA encoding a peptide comprising the sequence from aa 81 (Met) to aa 92 (Phe) in the amino acid sequence of SEQ ID NO: 23, the DNA comprising the sequence from base 241 to base 276 in the base sequence of SEQ ID NO: 24;
(25) For the DNA encoding a peptide comprising the sequence from aa 101 (Ser) to aa 112 (Leu) in the amino acid sequence of SEQ ID NO: 23, the DNA comprising the sequence from base 301 to base 336 in the base sequence of SEQ ID NO: 24;
(26) For the DNA encoding a peptide comprising the sequence from aa 95 (Asn) to aa 112 (Leu) in the amino acid sequence of SEQ ID NO: 23, the DNA comprising the sequence from base 283 to base 336 in the base sequence of SEQ ID NO: 24;
(27) For the DNA encoding a peptide comprising the sequence from aa 124 (Val) to aa 131 (Phe) in the amino acid sequence of SEQ ID NO: 23, the DNA comprising the sequence from base 370 to base 393 in the base sequence of SEQ ID NO: 24;
(28) For the DNA encoding a peptide comprising the sequence from aa 1 (Met) to aa 92 (Phe) in the amino acid sequence of SEQ ID NO: 23, the DNA comprising the sequence from base 1 to base 276 in the base sequence of SEQ ID NO: 24;
(29) For the DNA encoding a peptide comprising the sequence from aa 1 (Met) to aa 131 (Phe) in the amino acid sequence of SEQ ID NO: 23, the DNA comprising the sequence from base 1 to base 393 in the base sequence of SEQ ID NO: 24;
(30) For the DNA encoding a peptide comprising the sequence from aa 58 (Ser) to aa 94 (Phe) in the amino acid sequence of SEQ ID NO: 25, the DNA comprising the sequence from base 172 to base 282 in the base sequence of SEQ ID NO: 26;
(31) For the DNA encoding a peptide comprising the sequence from aa 83 (Val) to aa 94 (Phe) in the amino acid sequence of SEQ ID NO: 25, the DNA comprising the sequence from base 247 to base 282 in the base sequence of SEQ ID NO: 26;
(32) For the DNA encoding a peptide comprising the sequence from aa 84 (Pro) to aa 94 (Phe) in the amino acid sequence of SEQ ID NO: 25, the DNA comprising the sequence from base 250 to base 282 in the base sequence of SEQ ID NO: 26;
(33) For the DNA encoding a peptide comprising the sequence from aa 118 (Phe) to aa 125 (Phe) in the amino acid sequence of SEQ ID NO: 25, the DNA comprising the sequence from base 352 to base 375 in the base sequence of SEQ ID NO: 26;
(34) For the DNA encoding a peptide comprising the sequence from aa 58 (Ser) to aa 94 (Phe) in the amino acid sequence of SEQ ID NO: 27, the DNA comprising the sequence from base 172 to base 282 in the base sequence of SEQ ID NO: 28;
(35) For the DNA encoding a peptide comprising the sequence from aa 84 (Pro) to aa 94 (Phe) in the amino acid sequence of SEQ ID NO: 27, the DNA comprising the sequence from base 250 to base 282 in the base sequence of SEQ ID NO: 28.

The protein or polypeptide having cysteine at its N-terminus can be used in the method of the invention without a particular limitation. A protein or polypeptide not having cysteine at its N-terminus may also be used after modification so that it will have cysteine at its N-terminus by an ordinary method.

The protein or polypeptide having a cysteine at the N-terminal preferably has a molecular weight of 100 to 100,000, and more preferably a molecular weight of 300 to 50,000. In addition, the protein or polypeptide having a cysteine at the N-terminal preferably has 1 to 1,000 amino acids, and more preferably 3 to 500 amino acids.

The above-mentioned protein or peptide includes, for example, interferons, interleukins, various growth factors such as fibroblast growth factor (aFGF, bFGF, etc.), (pro)urokinase, lymphotoxin, tumor necrosis factor (TNF), enzyme proteins such as β-galactosidase, storage proteins, streptoavisine, protein A, protein G, tissue plasminogen activator (TPA), or muteins or parts (fragments) thereof.

Among those, fibroblast growth factor (aFGF, bFGF, etc.), or a mutein thereof, or a part thereof (a fragment) (for example, bFGF CS23 mutein, etc.) is preferably used.

An example of a bFGF CS23 mutein is a protein that comprises the following amino acid sequence (SEQ ID NO. 13):

Pro-Ala-Leu-Pro-Glu-Asp-Gly-Gly-Ser-Gly-Ala-Phe-Pro-Pro-Gly-His-
Phe-Lys-Asp-Pro-Lys-Arg-Leu-Tyr-Cys-Lys-Asn-Gly-Gly-Phe-Phe-Leu-
Arg-Ile-His-Pro-Asp-Gly-Arg-Val-Asp-Gly-Val-Arg-Glu-Lys-Ser-Asp-
Pro-His-Ile-Lys-Leu-Gln-Leu-Gln-Ala-Glu-Glu-Arg-Gly-Val-Val-Ser-
Ile-Lys-Gly-Val-Ser-Ala-Asn-Arg-Tyr-Leu-Ala-Met-Lys-Glu-Asp-Gly-

Arg-Leu-Leu-Ala-Ser-Lys-Ser-Val-Thr-Asp-Glu-Cys-Phe-Phe-Phe-Glu-
Arg-Leu-Glu-Ser-Asn-Asn-Tyr-Asn-Thr-Tyr-Arg-Ser-Arg-Lys-Tyr-Thr-
Ser-Trp-Tyr-Val-Ala-Leu-Lys-Arg-Thr-Gly-Gln-Tyr-Lys-Leu-Gly-Ser-
Lys-Thr-Gly-Pro-Gly-Gln-Lys-Ala-Ile-Leu-Phe-Leu-Pro-Met-Ser-Ala-
Lys-Ser.

For the DNA encoding the fusion protein (including fusion polypeptides) used in the method of the invention, (1) the entire nucleic acid sequence of said DNA may be chemically synthesized; or (2) said DNA may be constructed by adding the nucleic acid sequence encoding cysteine to the N-terminal of the nucleic acid sequence encoding a protein, and then adding the nucleic acid sequence encoding the polypeptide of the invention to the N-terminal. Moreover, (3) to obtain a fragment of the polypeptide of the invention, said DNA may be constructed using a technique such as site-directed mutagenesis for substituting the amino acid residue just after the desired fragment with cysteine.

In the above producing method (1), well-known methods, for example, phosphoamidide method, phosphotriester or diester method, or hydrogen phosphonate method may be used. The DNA of shorter length may be synthesized completely at once, and the DNA of longer length may be produced by synthesizing parts and ligating the parts using T4DNA ligase.

In the above producing method (2), the DNA encoding the protein on the C-terminal side is obtained by excising it from chromosomes with a suitable restriction enzyme and then subcloning it into a vector; or its cDNA is obtained. Subsequently, the DNA is cleaved with an appropriate restriction enzyme so that the N-terminal amino acid is cysteine, or a synthetic DNA is ligated to 5'-terminal of the DNA encoding the protein or a part thereof so that the N-terminal amino acid is cysteine after the modification. Finally, a DNA encoding the objective protein (chemically synthesized, or cloned from an organism) is ligated to the 5'-terminal.

Examples of the DNA encoding the fusion protein obtained in the above manner include a DNA represented by the following formula: 
AGCCTGAACTTTGAAGAACTGAAAGATTGGGGTCCGAAAAATGTGATTAAAATGAGCACCCCGGCG
GTGAATAAAATGCCGCATAGCTTTGCGAATCTGCCGCTGCGTTTT-TGC or TGT-R (I)
 wherein, R indicates the following nucleic acid sequence (SEQ ID NO: 14, a fragment of bFGFCS23 mutein):

CCCGAGGATGGCGGCAGCGGCGCCTTCCCGCCCGGCCACTTCAAGGAC
CCCAAGCGGCTGTACTGCAAAAACGGGGGCTTCTTCCTGCGCATCCACCCCGACGGCCGA
GTTGACGGGGTCCGGGAGAAGAGCGACCCTCACATCAAGCTACAACTTCAAGCAGAAGAG
AGAGGAGTTGTGTCTATCAAAGGAGTGAGCGCTAATCGTTACCTGGCTATGAAGGAAGAT
GGAAGATTACTAGCTTCTAAGTCTGTTACGGATGAGTGTTTCTTTTTTGAACGATTGGAA
TCTAATAACTACAATACTTACCGGTCAAGGAAATACACCAGTTGGTATGTGGCACTGAAA
CGAACTGGGCAGTATAAACTTGGATCCAAAACAGGACCTGGGCAGAAAGCTATACTTTTT
CTTCCAATGTCTGCTAAGAGCTGC.

The above formula (1) means that the nucleic acid sequence represented by R is ligated to the DNA sequence encoding a polypeptide comprising the amino acid sequence of SEQ ID NO: 7 through the nucleic acid sequence encoding cysteine.

The DNA (plasmid) having ATG on the 5'-terminal, a downstream region encoding the fusion protein and a translation termination codon, can be produced by chemical synthesis or by processing a cDNA encoding the well-known protein, which is prepared by genetic engineering, or by processing a DNA encoding the protein, which is derived from chromosomes.

In the present invention, the DNA encoding the fusion protein or polypeptide, in which the polypeptide of the invention is attached to the N-terminal of a protein or polypeptide having a cysteine at the N-terminal, can be modified by using conventional DNA technology; for example, site-directed mutagenesis technology, so that it encodes the desired mutein.

Site-directed mutagenesis technology is well known, and is described in R.F. Lather and J.P. Lecoq: Genetic Engineering, Academic Press (1983), p. 31-50. Oligonucleotide-directed mutagenesis is described in M. Smith and S. Gillam: Genetic Engineering:
Principles and Methods, Plenum Press (1981), Vol. 3, p. 1-32.

To produce a plasmid comprising a DNA region encoding the fusion protein or polypeptide of the invention, in which the polypeptide of the invention is linked to the N-terminal of a protein or polypeptide having a cysteine at the N-terminal (hereinafter referred to as the fusion protein of the invention), a plasmid vector derived from Escherichia Coli may be used: for example, pBR322 (Gene 2, 95(1977)), pBR313 (Gene 2, 75(1977)), pBR324, pBR325 (Gene 4, 124(1978)), pBR327, pBR328 (Gene 9, 287(1980)), pBR329 (Gene 17, 79(1982)), pKY2289 (Gene 3, 1(1978)), pKY2700 (Biochemistry 52, 770(1980)), pACYC177, pACYC184 (Journal of Bacteriology, 134, 1141 (1978)), pRK248, pRK646, pDF (Methods in Enzymology, 68, 268(1979)), pUC18, and pUC19 (Yanischperon, et al: Gene, 33, 103(1985)). In addition, bacteriophage vectors may also be used: for example, λ-phage vectors, e.g. λgt-vectors such as λgt-λC (Proc. Natl. Acad. Sci. U.S.A. 71, 4579(1974)), λgt-λB (Proc. Natl. Acad. Sci. U.S.A. 72, 3461(1975)), λDam (Gene, 1, 255(1977)), and Charon vector (Science, 196, 161(1977); Journal of Virology, 29, 555(1979)); and filamentous phage vectors, e.g. mp-vectors such as mp18, mp19 (Yanishperon et al, Gene, 33, 103(1985).

The said DNA preferably has a promoter upstream of the ATG, and said promoter may be any promoter suitable for the host used in the production of a transformant.

For example, for Escherichia coli, trp promoter, lac promoter, recA promoter, λPL promoter, lpp promoter, and T7 promoter, etc.; for Bacillus subtilis, SP01 promoter, SP02 promoter, and penP promoter; for Saccharomyces cerevisiae, PHO5 promoter, PGK promoter, GAP promoter, and ADH promoter; and for animal cells, a promoter derived from SV40, may be used. The SD (Shine and Delgarno) sequence may be inserted downstream of the promoter, if necessary.

When using T7 promoter system, any of 17 different T7 promoters located on T7 DNA (J.L. Oakley et al.,: Proc. Natl. Acad. Sci. U.S.A., 74: 4266-4270 (1977), M. D. Rosa, Cell, 16: 815-825 (1979), N. Panayotatos, et al., Nature, 280 : 35 (1979), J.J. Dunn et al., J. Mol. Biol., 166: 477-535 (1983)) may be used as the T7 promoter. φ10 promoter (A.H. Rosenberg et. al., Gene, 56: 125-135 (1987) is preferred.

As the transcription terminator, a terminator that functions in Escherichia coli strains, preferably Tφ terminator (F.W. Studier et. al., J. Mol. Biol., 189: 113-130 (1986)) may be used.

A gene of T7 RNA polymerase may be T7 Gene (F.W. Studier et. al., J. Mol. Biol., 189: 113-130(1986)).

The preferred vector is one constructed by incorporating T7 promoter and T7 terminator into the aforementioned vector. Such a vector includes pET-1, pET-2, pET-3, pET-4, pET-5 (A. H. Rosenberg, Gene 56: 125-135 (1987), and pTB960-2 (EP-A-499990)). pTB960-2 is preferably used.

The transformant of the invention can be produced by transforming a host with the expression plasmid obtained by the aforementioned method, according to a well-known method (e.g. S.N. Cohen, et al, Proc. Natl. Acad. Sci. U.S.A., 69, 2110 (1972)).

Microorganism hosts to be transformed include Escherichia bacteria, Bacillus bacteria, yeast, and animal cells.

Said Escherichia bacteria include Escherichia coli (E. coli), specifically, E.coli K12DH1 (Proc. Natl. Acad. Sci. U.S.A., 60, 160 (1968)), JM-103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), N4830 (Cell, 25, 713 (1981)), K-12 MM294 (Proc. Natl. Acad. Sci. U.S.A., 73, 4174 (1976)), and BL-21.

Said Bacillus bacteria include Bacillus subtilis, specifically, Bacillus subtilis MI114 (Gene, 24, 255 (1983)), and 207-21 (Journal of Biochemistry, 95, 87 (1984)).

Said yeast includes Saccharomyces cerevisiae, specifically, Saccharomyces cerevisiae AH22 (Proc. Natl. Acad. Sci. U.S.A., 75, 1929 (1978)), XSB5 2-23C(Proc. Natl. Acad. Sci. U.S.A., 77, 2173 (1980)), BH-641A (ATCC 28339), 20B-12 (Genetics, 85, 23 (1976)), and GM3C-2 (Proc. Natl. Acad. Sci. U.S.A., 78, 2258 (1981)).

Said animal cells includes Monkey cell COS-7 (Cell, 23, 175 (1981)), Vero (Nihon Rinshou [Japan Clinic] 21, 1209 (1963)), Chinese hamster cells CHO (Journal of Experimental Medicine, 108, 945 (1985)), mouse L cells (Journal of National Cancer Institute, 4, 165 (1943)), human FL cells (Proceedings of the Society of Experimental Biology and Medicine, 94, 532 (1957)), and hamster C cells.

When utilizing T7 promoter system, an E.coli strain having T7 RNA polymerase gene (T7 gene 1) (F.W. Studier et al., Journal of Molecular Biology, 189, 113-130 (1986)) may be used as the host for transformation, including E.coli MM294, DH-1, C600, JM109, BL21. Alternatively, an E.coli strain having T7 RNA polymerase gene (T7 gene 1) in combination with another plasmid may also be used. Preferably, MM294 strain or BL21 strain is used, in which a λ phage having T7 gene 1 is lysogenized. In this case, the promoter for T7 gene 1 may be lac promoter, under which the expression is induced by isopropyl-1-thio-β-D-galactopyranoside (IPTG).

The transformation of Bacillus bacteria hosts can be carried out according to a well-known method, such as one described in Molecular and General Genetics, 168, 111 (1979).

The transformation of yeast hosts can be carried out according to a well-known method, such as one described in Proc. Natl. Acad. Sci. U.S.A. 75, 1929 (1978).

The transformation of animal cell hosts can be carried out according to a well-known method, such as one described in the Virology, 52, 456 (1973).

The fusion protein of the invention can be produced by culturing the above-described transformant in a medium, and collecting the fusion protein produced.

It is desirable that the culture medium has pH of approximately 6 to 8.

For example, M9 medium containing glucose and casamino acid (Journal of Experiments in Molecular Genetics, 431-433, Cold Spring Harbor Laboratory, New York, 1972) is preferable as the medium for culturing Escherichia bacteria. An agent such as 3β-indolyl acrylate or isopropyl β-D-thiogalactopyranside (IPTG) may be added to the medium in order to increase the efficiency of the promoter, if necessary.

When the host is Escherichia bacteria, it is normally cultured at approximately 15 to 43° C for approximately 3 to 24 hours, and aeration and agitation may be applied if necessary.

When the host is Bacillus bacteria, it is normally cultured at approximately 30 to 40° C for approximately 6 to 24 hours, and aeration and agitation may be applied if necessary.

When culturing a yeast transformant, for example, Burkholder minimum medium (Bostian, K.L. et al., Proc. Natl. Acad. Sci. U.S.A., 77, 4505 (1980)) may be used. It is preferable to adjust the pH of the culture medium to approximately 5 to 8. Such a culture is normally conducted at approximately 20 to 35° C for approximately 24 to 72 hours, and aeration and agitation may be applied if necessary.

When culturing an animal cell transformant, MEM medium (Science, 122, 501 (1952)), DME medium (Virology, 8, 396 (1959)), RPMI 1640 medium (The Journal of the American Medical Association, 199, 519 (1967)), and 199 medium (Proceedings of the Society for Biological Medicine, 73, 1 (1950)) containing approximately 0.2 to 20%, preferably approximately 5 to 20% fetal bovine serum may be used. The pH is preferably approximately 6 to 8. Such a culture is normally conducted at approximately 30 to 40° C for approximately 15 to 60 hours, and aeration and agitation may be applied if necessary.

The fusion protein of the invention can be produced by culturing the aforementioned transformant to express and accumulate the fusion protein in the culture medium, and then harvesting it.

M9 medium including glucose and casamino acid (J. Miller, Experiments in Molecular Genetics, 431-433 (Cold Spring Harbor Laboratory, New York 1972)), 2x YT medium (Messing, Methods in Enzymology, 101, 20 (1983)), and LB medium may be used for the culture medium.

Such a culture is normally conducted at approximately 15 to 43° C for approximately 3 to 24 hours, and aeration and agitation may be applied if necessary.

When using a recombinant cell having a λcI-ts repressor and an expression vector having λPL-promoter, it is cultured at a temperature of 15 to 36° C, preferably approximately 30 to 36° C, and it is preferable to inactivate the λcI-ts repressor at approximately 37 to 42° C. In addition, in order to enhance the efficiency of the recA promoter, i.e. to reduce the function of inhibiting the recA gene expression, optionally, agents such as mitomycin C and nalidixic acid may be added, the culture may be exposed to ultraviolet light, or the pH of the culture medium may be changed to alkali side.

When using T7 promoter system, (1) IPTG, etc. is added to express T7 gene (RNA polymerase gene) ligated downstream of the lac promoter; or (2) the temperature of the culture medium is increased to express T7 gene (RNA polymerase. gene) ligated downstream of the λPL-promoter. As the result, the expressed T7 phage RNA polymerase 1 acts on the T7 promoter in a specific manner.

After the culture is finished, for example, the bacteria are collected by a well-known method, suspended in a buffer solution, and then crushed by treatment with a protein-denaturing agent, ultrasonication, enzymes such as lysozyme, glass beads, French press, or freeze-thawing. The supernatant is obtained by a well-known method, such as centrifugation.

Conventional protein purification methods may be employed to isolate the fusion protein from the supernatant obtained above. An appropriate combination of, for example, gel filteration, ion-exchange chromatography, absorption chromatography, high speed liquid chromatography, affinity chromatography, hydrophobic chromatography, and electrophoresis may be used.

When the N-terminal of the polypeptide of the invention is Isoleucine, Valine, Cysteine, Threonine, Aspartic acid, Lysine, Leucine, Arginine, Asparagine, Methionine, Phenylalanine, Tyrosine, Tryptophan, Glutamic acid, Glutamine or Histidine (for example, when the polypeptide is one having the amino acid sequence shown in SEQ ID NO: 3, 5, 9 or 11), there are cases in which methionine derived from the translation initiation codon is added to the N-terminal of the fusion protein obtained as described above.

Alternatively, the fusion protein may be submitted to the following reaction step without being purified or in a partially purified state.

Subsequently, the fusion protein of the invention thus obtained is submitted to a reaction for cleavage of the peptide bond on the amino-terminal side of the cysteine residue.

This cleavage reaction may be, for example, S-cyanation reaction, which is followed by a hydrolysis reaction.

When an amide form of the polypeptide or a salt thereof is desired as the final product, the S-cyanation reaction is followed by ammonolysis for the cleavage reaction.

The S-cyanation reaction is conducted by reacting an S-cyanation reagent with the source material.

The S-cyanation reagent includes 2-nitro-5-thiocyanobenzoic acid (NTCB), 1-cyano-4-dimethyl aminopyridinium salt (DMAP-CN), and CN⁻ ions. The used amount of the S-cyanation reagents may be approximately 2 to 50 times to the total amount of thiol group, and preferably approximately 5 to 10 times.

The reaction temperature may be between approximately 0 and 80°C, and preferably between approximately 0 and 50°C. Any buffer solution may be used as the solvent as long as it does not react with the S-cyanation reagent, including tris-hydrochloride buffer solution, tris-acetate buffer solution, phosphate buffer solution, and borate buffer solution. An organic solvent may be further contained as long as it does not react with the S-cyanation reagent.

The cleavage reaction may be carried out at pH of 1 to 12. In particular, pH 7 to 10 is preferable when using NTCB, and pH 2 to 7 is preferable when using DMAP-CN in order to prevent an S-S exchange reaction. The reaction solution may further contain a denaturing agent such as guanidine hydrochloride.

Alkali treatment may be carried out for the aforementioned hydrolysis reaction.

The alkali treatment is conducted by adjusting the pH of an aqueous solution containing the source material to 7 to 14.

For the pH adjustment, a suitable amount of a solution of ammonia, sodium hydroxide, amino compound, trizma base (tris(hydroxymethyl)-aminomethane), sodium II phosphate, potassium hydroxide, or barium hydroxide is added to the aqueous solution containing the source material. In particular, ammonia solution is preferable.

For the aforementioned treatment, the following concentration of each solution may be used: approximately 0.01 to 15 N, preferably approximately 0.1 to 5 N, for ammonia or amino compound; approximately 0.01 to 2 N, preferably approximately 0.05 to 1 N, for sodium hydroxide; approximately 1 mM to 1 M, preferably approximately 20 mM to 200 mM, for trizma base; approximately 1 mM to 1 M, preferably approximately 10 mM to 100 mM, for sodium II phosphate; approximately 0.01 to 4 N, preferably approximately 0.1 to 2 N, for potassium hydroxide; approximately 0.01 to 0.2 M, preferably approximately 0.1 to 0.2 M, for potassium hydroxide. The reaction temperature may be between approximately -20 and 80 °C, and preferably between approximately -10 and 50 °C.

The reaction time for S-cyanation reaction may be approximately 1 to 60 min., preferably approximately 15 to 30 min.; for ammonolysis, approximately 5 min. to 24 hours, preferably approximately 10 to 180 min.; and for hydrolysis reaction, approximately 5 min. to 100 hours, preferably approximately 10 min. to 15 hours.

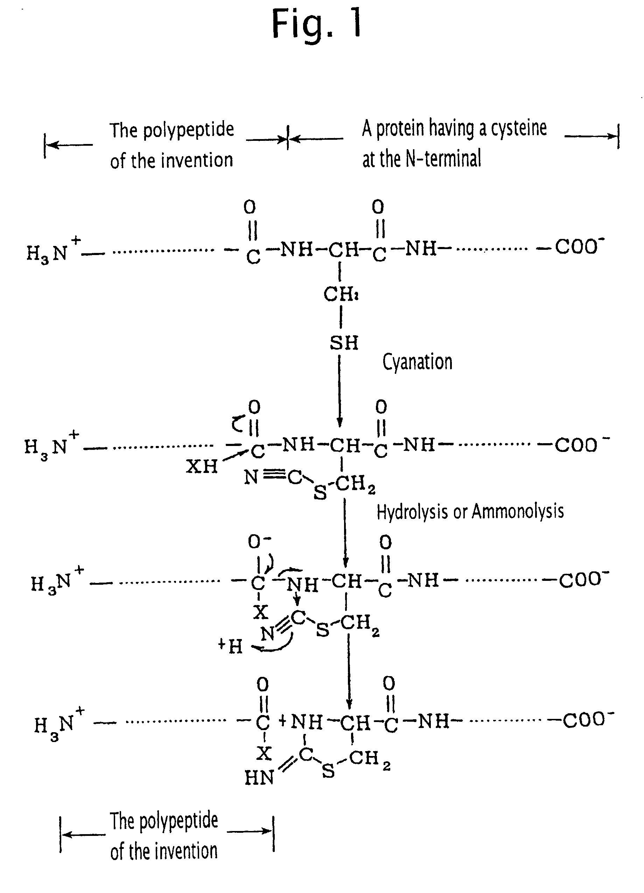

The S-cyanation reaction or the hydrolysis reaction may occur as indicated in Figure 1. In Figure 1, X represents R¹-(NR²)- or OH, wherein R¹ and R² may be the same or different, and each indicates (i) hydrogen; (ii) C₁₋₂₀ alkyl, C₃₋₈ cycloalkyl, C₆₋₁₄ aryl, or C₆₋₁₄ aryl-C₁₋₃ alkyl (which may be unsubstituted or substituted with 1 to 3 amino groups or hydroxyl groups on a carbon atom); (iii) amino optionally substituted; (iv) hydroxyl or C₁₋₆ alkoxy.

The aforementioned C₁₋₂₀ alkyl indicates, for examples, methyl, ethyl, propyl, isopropyl, butyl, sec-butyl, penthyl, isopenthyl, neopenthyl, 1- ethylpenthyl, hexyl, isohexyl, heptyl, octyl, nonanyl, decanyl, undecanyl, dodecanyl, tetradecanyl, pentadecanyl, hexadecanyl, heptadecanyl, octadecanyl, nonadecanyl, and eicosanyl.

The aforementioned C₃₋₈ cycloalkyl indicates, for examples, cyclopropyl, cyclobutyl, cyclopenthyl, cyclohexyl, cycloheptyl, and cyclooctyl.

The aforementioned C₆₋₁₄ aryl indicates, for examples, phenyl, naphthyl, anthryl, phenanthryl, and acenaphthylenyl.

The aforementioned C₆₋₁₄ aryl-C₁₋₃-alkyl indicates, for examples, benzyl, phenethyl, 3-phenylpropyl, (1-naphthyl)methyl, and (2-naphthyl)methyl.

The aforementioned C₁₋₆ alkoxy indicates, for examples, methoxy, ethoxy, propoxy, butoxy, penthyloxy, and hexyloxy.

Substituents for the amino group optionally substituted, described in (iii) above, include amino acids, and peptides having 2 to 10 amino acids.

The above amino acids may be L-form or D-form, including Ala, Arg, Asp, Asn, Glu, Gin, Gly, His, lle, Met, Leu, Phe, Pro, Ser, Thr, Trp, Tyr, and Val.

When using ammonia or an amino compound in the reaction, the corresponding amide form is obtained.

In general, known methods for peptide purification may be used to isolate the excised polypeptide of the invention. An appropriate combination of, for example, gel filtering, ion-exchange chromatography, high speed liquid chromatography, affinity chromatography, hydrophobic chromatography, thin layer chromatography or electrophoresis may be used.

As described above, there is a case where a methionine derived from the translation initiation codon is added to the N-terminal of the polypeptide of the invention. In this case, it is possible to remove the N-terminal methionine, for example, by a reaction with an α-diketone such as glyoxylic acid (preferably, in the presence of a transition metal ion such as copper sulfate, and a base such as pyridine); and a subsequent hydrolysis with a diamine such as o-phenylenediamine. Such a method is described in detail below.

### [The method of removing the N-terminal methionine from the polypeptide of the invention]

In the specification, the "methionine residue optionally oxidized" indicates a methionine residue or its S-oxidized form. The S-oxidized form of methionine residue includes a sulfoxide or sulfone thereof.

The polypeptide of the invention having the methionine residue optionally oxidized at the N-terminal includes a polypeptide represented by:
the formula

CH₃-S(O)ₘ-(CH₂)₂-CO-X

wherein m indicates an integer of 0 to 2, and X indicates a peptide chain of the polypeptide of the invention.

The polypeptide may be in a salt form, including the same salt forms as those of the polypeptide of the invention as described above.

The polypeptide of the invention having the methionine residue optionally oxidized at the N-terminal, or a salt thereof is preferably one produced by the genetic engineering.

In the said formula, m is preferably 0. The peptide chain of the polypeptide of the invention as indicated by X includes the polypeptide of the invention as described above.

The said polypeptide of the invention or a salt thereof may be refolded (activated, renatured) after or before being subjected to the process of removing the N-terminal methionine residue optionally oxidized or a diketone of the methionine residue.

In the specification, the polypeptide of the invention having a diketone of the methionine residue optionally oxidized at the N-terminal, or a salt thereof indicates a compound represented by:
the formula

CH₃-S(O)ₘ-(CH₂)₂-CO-CO-X

wherein m indicates an integer of 0 to 2, and X indicates a peptide chain of the polypeptide of the invention,
or a salt thereof. The polypeptide of the invention having a diketone of the methionine residue optionally oxidized at the N-terminal, or a salt thereof can be produced through a variety of reactions such as a chemical reaction and an enzyme reaction. For example, the polypeptide of the invention having a diketone of the methionine residue optionally oxidized at the N-terminal, or a salt thereof can be produced by a transamination reaction, in which the polypeptide of the invention having a methionine residue optionally oxidized at the N-terminal, or a salt thereof is reacted with an α-diketone.

In the specification, the α-diketone may be any one capable of carrying out the transamination of the said polypeptide of the invention or a salt thereof, and includes a compound represented by:
the formula

R1-CO-CO-R2

wherein R1 indicates a hydrogen, or a lower alkyl or phenyl group, which may be optionally substituted with a carboxyl group (preferably hydrogen or methyl; more preferably hydrogen); and R2 indicates a hydroxyl, a lower alkoxy, or an amino group optionally substituted with a lower alkyl (preferably hydroxy),
or a salt thereof.

In the said formula, the lower alkyl group represented by R1 includes a C₁₋₆-alkyl such as methyl, ethyl, propyl, i-propyl, butyl, i-butyl, sec-butyl, t-butyl. The lower alkoxy group represented by R2 includes a C₁₋₆-alkoxy such as methoxy, ethoxy, propoxy, i-propoxy, butoxy, i-butoxy, sec-butoxy, t-butoxy. The amino group optionally substituted with a lower alkyl indicates an amino group, which may have one or two lower alkyl groups as represented by R1. In addition, the said salt form includes the same salt forms as described regarding the salts of the polypeptide of the invention having the methionine residue optionally oxidized at the N-terminal.

Examples of the α-diketone include glyoxylic acid, pyruvic acid, oxalacetic acid, phenylglyoxylic acid, and 2-oxoglutaric acid. Among them, glyoxylic acid is preferably used.

The α-diketone may be in a salt form, including an alkali metal salt such as a sodium salt, a potassium salt; and a salt with an inorganic acid such as a hydrochloride.

In the transamination reaction, it is generally preferred that the polypeptide of the invention having a methionine residue optionally oxidized at the N-terminal, or a salt thereof is reacted with an α-diketone in an amount of about 1 to 10000 moles (preferably 2000 to 4000 moles) of the α-diketone per mole of the polypeptide of the invention or a salt thereof at a temperature between 0 and 70 °C (preferably between about 20 and 40 °C) for a time period between about 10 min and 5 hr (preferably between about 20 min and 2 hr). Any buffer solution may be used, which does not inhibit the transamination, including phosphate buffer, acetate buffer, and citrate buffer. In particular, an acetate buffer solution is preferred. The pH of the reaction solution is adjusted to about 2 to 9, preferably about 4 to 7, in particular, about 5 to 6 to achieve the reaction.

It is preferred that the reaction with an α-diketone is carried out in the presence of a transition metal ion to enhance the transamination. Generally, 0.001 to 0.1 mole (preferably 0.01 to 0.05 mole) of the transition metal ion per mole of α-diketone is used. Examples of the transition metal ion include a copper ion (Cu+, Cu2+), cobalt ion (Co2+, Co3+), nickel ion (Ni2+, Ni3+), iron ion (Fe2+, Fe3+), zinc ion (Zn2+), aluminum ion (A13+), manganese ion (Mn2+, etc), gallium ion (Ga3+), indium ion (In3+), magnesium ion (Mg2+), and calcium ion (Ca2+). Among these, preferably copper ion and cobalt ion; more preferably copper ion is used. The transition metal ion may be added to the reaction medium in a salt form with an inorganic acid, such as sulfuric acid, nitric acid, hydrochloric acid, or perchloric acid, or in a salt form with an organic acid, such as acetic acid, oxalic acid, citric acid, or carbonic acid. Among these, preferably copper sulfate and copper acetate; more preferably copper sulfate is used.

In addition, it is preferred that the reaction with an α-diketone is carried out in the presence of a base. Generally, 0.1 to 20 moles (preferably 0.5 to 10 moles) of the base per mole of α-diketone are used. Examples of the base include an alkyl amine such as triethylamine, tributylamine, and an aromatic amine such as N,N-dimethylaniline, pyridine, lutidine, collidine, 4-(dimethylamino) pyridine, and imidazole. Among these, pyridine is preferably used.

In the transamination reaction, it is preferred that urea is further contained in the buffer solution for the transamination in order to prevent the precipitation of the polypeptide of the invention having a methionine residue optionally oxidized at the N-terminal or a salt thereof, and also the polypeptide of the invention having a diketone of the methionine residue optionally oxidized at the N-terminal or a salt thereof, which is produced by the transamination of the polypeptide of the invention having the methionine residue. For example, urea is added to the buffer solution preferably at a final concentration of about 1 to 8 M, more preferably about 3 to 6 M.

Furthermore, in the transamination reaction, it is preferred that the reaction with an α-diketone is carried out in the presence of a transition metal ion and a base. In practice, a mixture containing the three components, i.e. a transition metal ion, a base and an α-diketone (for example, copper sulfate, pyridine and glyoxylic acid) is added to the aqueous solution containing the polypeptide of the invention having a methionine residue optionally oxidized at the N-terminal or a salt thereof to achieve the transamination.

The compound or a salt thereof, produced by the transamination reaction and represented by:
the formula

CH₃-S(O)ₘ-(CH₂)₂-CO-CO-X

wherein m indicates an integer of 0 to 2, and X indicates a peptide chain of the polypeptide of the invention,
(i.e. the polypeptide of the invention having a diketone of the methionine residue optionally oxidized at the N-terminal or a salt thereof) can be isolated and purified from the reaction solution by well-known peptide/protein purification techniques, for example, extraction, salting out, distribution, re-crystallization, or chromatography, before it is subjected to a hydrolysis reaction. Alternatively, the compound may be subjected to the hydrolysis reaction as it is without being purified.

The polypeptide of the invention having a diketone of the methionine residue optionally oxidized at the N-terminal or a salt thereof, which is produced by the transamination, can be generally subjected to the hydrolysis reaction with a base, so that it can be changed to the polypeptide of the invention not having the methionine residue optionally oxidized or the diketone thereof at the N-terminal, or a salt thereof.

Examples of the base used in the hydrolysis reaction include an amine and a salt thereof, for example, an alkyl amine such as cysteamine, triethylamine, tributylamine, and salts thereof; an aromatic amine such as N,N-dimethylaniline, pyridine, lutidine, collidine, 4-(dimethylamino) pyridine, imidazole, and salts thereof; a diamine such as o-phenylenediamine, tolylene-3,4-diamine, 3,4-diamino-benzoic acid, N-alkyl-substituted forms thereof (e.g. N-methyl-1,2- phenylenediamine, N-ethyl-1,2-phenylenediamine, N-isopropyl-1,2- phenylenediamine), 2,3-diaminophenol, 4-chloro-o-phenylenediamine (preferably an aromatic diamine, especially 3,4-diamino-benzoic acid and N-alkyl-substituted forms thereof (e.g. N-methyl-1,2- phenylenediamine, N-ethyl-1,2-phenylenediamine, N-isopropyl-1,2- phenylenediamine)), and salts thereof; a thiosemicarbazide such as thiosemicarbazide, acetone thiosemicarbazide, phenyl thiosemicarbazide; and a selenosemicarbazide such as selenosemicarbazide, acetone selenosemicarbazide. Among these, an amine, especially a diamine or a thiosemicarbazide, or a salt thereof is preferably used. In particular, 3,4-diamino-benzoic acid and a salt thereof are preferred.

The salt of the base used in the hydrolysis reaction includes the same salt forms as described regarding the salts of the polypeptide of the invention having the methionine residue optionally oxidized at the N-terminal.

The amount of the base is usually about 1 to 10000 moles, preferably about 200 to 3000 moles, and more preferably about 500 to 3000 moles per mole of the polypeptide of the invention having the diketone of the methionine residue or a salt thereof. The hydrolysis reaction is usually carried out at a temperature between 0 and 70 °C (preferably between about 20 and 40 °C) for a time period between about 1 hr and 7 days (preferably between about 10 hrs and 5 days). A buffer solution is preferably used as a medium in the reaction, including phosphate buffer, acetate buffer, citrate buffer, and formate buffer. Any buffer solution may be used, which does not inhibit the hydrolysis reaction. In particular, preferred are the formate buffers, such as acetic acid/sodium formate buffer, formic acid/sodium formate buffer, and formic acid/sodium acetate buffer. The pH of the reaction solution is adjusted to about 2 to 9, preferably about 3 to 7, in particular, about 4 to 6 to achieve the reaction. Such a buffer is preferably used at a concentration of about 0.1 to 8 mol/L, more preferably about 0.5 to 6 mol/L.

In the hydrolysis reaction, it is preferred that urea is further contained in the buffer solution for the hydrolysis in order to prevent the precipitation of the polypeptide of the invention having a diketone of the methionine residue optionally oxidized at the N-terminal or a salt thereof, and also the polypeptide of the invention not having the methionine residue or a salt thereof, which is produced by the hydrolysis reaction. For example, urea is added to the buffer solution preferably at a final concentration of about 1 to 6 M, more preferably about 2 to 5 M.

The polypeptide or its salt of the invention obtained in this way may also be isolated and purified from the reaction solution by well-known purification techniques, for example, extraction, salting out, distribution, re-crystallization, or chromatography. The preferable purification method is ion-exchange chromatography using SP-sepharose (Pharmacia Biotech, Co., Ltd.) or DEAE-5PW (Tosoh Co., Ltd.).

The polypeptide or its salt of the invention produced by the method of the invention may be formulated with sterilized water, human serum albumin (HSA), physiological saline and other well-known physiologically acceptable carriers, and then may be administered to a mammal (e.g. human) parenterally or locally. For example, the polypeptide or its salt of the invention may be administered parenterally by intravenous or intramuscular injection in a daily dose per person of approximately 0.01 to 50 mg, preferably approximately 0.1 to 10 mg.

The formulation comprising the polypeptide or its salt of the invention produced by the method of the invention may further contain salts, diluents, adjuvants, other carriers, buffers, binders, surfactants, preservatives, and other physiologically acceptable active components. The parenteral dosage formulation may be provided as an ampoule of a suspension in a sterilized water solution or physiologically acceptable solvent, or as an ampoule of sterilized powder (normally obtained by lyophilization of the peptide solution) which can be diluted with a physiologically acceptable diluent at use.

The polypeptide of the invention produced by the method of the invention has an activity of regulating, i.e. promoting or inhibiting the prolactin secretion. On one hand, the polypeptide of the invention has an activity of promoting the prolactin secretion, and is thus useful as a prophylactic or therapeutic agent for various diseases associated with prolactin hypo-secretion. On the other hand, the polypeptide of the invention also shows an activity of inhibiting the prolactin secretion, which is mediated by the induction of desensitization of the prolactin secretion by administrating an increasing amount of the polypeptide having a strong affinity to its receptor protein. In this case, the polypeptide of the invention can be used as a prophylactic or therapeutic agent for various diseases associated with prolactin hyper-secretion.

Accordingly, the polypeptide of the invention is useful as an enhancer of the prolactin secretion or a prophylactic or therapeutic agent for various diseases relating to the prolactin secretion, e.g. hypoovarianism, seminal gland hypoplasia, osteoporosis, menopausal disorder, lactation failure, hypothroidism, renal failure.

In addition, the polypeptide of the invention is useful as an aphrodisiac because it has an aphrodisiac effect (pheromonal effect) based on its activity of inhibiting the prolactin secretion.

Meanwhile, the polypeptide of the invention is useful as an inhibitor of the prolactin secretion or a prophylactic or therapeutic agent for various diseases relating to the prolactin secretion, e.g. hyperprolactinemia, pituitary gland tumor, diencephalic tumor, menstrual disorder, stress, autoimmune disease, prolactinoma, infertility, impotence, amenorrhea, chylorrhea, acromegaly, Chiari-Frommel syndrome, Argonz-del Castilo syndrome, Forbes-Albright syndrome, breast carcinoma, lymphoma, Sheehan syndrome, or dysspermatogenesis.

In addition, the polypeptide of the invention is also useful as an anticonceptive, due to its activity of inhibiting the prolactin secretion.

Further, the polypeptide of the invention is also useful as an assay agent to examine the prolactin secretion activity, and as an animal drug, for example, a lactation enhancer in domestic mammals such as a cow, goat and pig, which is expected to be applied, for example, in the production of a utile material, where the utile material may be produced inside such a domestic mammal, and released into milk thereof.

Furthermore, the polypeptide of the invention has the activity of regulating placental function, and thus is also useful as a prophylactic or therapeutic agent for choriocarcinoma, hydatid mole, invasive mole, miscarriage, fetal dysgenesis, dysbolism of saccharide, dysbolism of lipid, or induction of delivery.

In the present specification and drawings, codes of amino acids, peptides, protecting groups, active groups, and other substances are denoted on the base of the IUPAC-IUB (Commission on Biochemical Nomenclature) or conventional codes used in the related field. Examples are shown below. In addition, when an amino acid can have optical isomers, unless specifically noted, the L-form is meant.
- DNA: Deoxyribonucleic acid
- A: Adenine
- T: Thymine
- G: Guanine
- C: Cytosine
- RNA: Ribonucleic acid
- EDTA: Ethylene diamine tetra acetic acid
- Gly: Glycine
- Ala: Alanine
- Val: Valine
- Leu: Leucine
- Ile: Isoleucine
- Ser: Serine
- Thr: Threonine
- Met: Methionine
- Glu: Glutamic acid
- Asp: Aspartic acid
- Lys: Lysine
- Arg: Arginine
- His: Histidine
- Phe: Phenylalanine
- Tyr: Tyrosine
- Trp: Tryptophan
- Pro: Proline
- Asn: Asparagine
- Gln: Glutamine
- ATP: Adenosine triphospate

The SEQ ID NOs. in the Sequence Listing indicate the following sequences.
[SEQ ID NO: 1]

This sequence shows the amino acid sequence of the polypeptide of the invention consisting of 9 amino acid residues.
[SEQ ID NO: 2]

This sequence shows the base sequence of the DNA encoding the polypeptide having the amino acid sequence of SEQ ID NO: 1.
[SEQ ID NO: 3]

This sequence shows the amino acid sequence of the polypeptide of the invention consisting of 12 amino acid residues.
[SEQ ID NO: 4]

This sequence shows the base sequence of the DNA encoding the polypeptide having the amino acid sequence of SEQ ID NO: 3.
[SEQ ID NO: 5]

This sequence shows the amino acid sequence of the polypeptide of the invention consisting of 20 amino acid residues.
[SEQ ID NO: 6]

This sequence shows the base sequence of the DNA encoding the polypeptide having the amino acid sequence of SEQ ID NO: 5.
[SEQ ID NO: 7]

This sequence shows the amino acid sequence of the polypeptide of the invention consisting of 37 amino acid residues.
[SEQ ID NO: 8]

This sequence shows the base sequence of the DNA encoding the polypeptide having the amino acid sequence of SEQ ID NO: 7.
[SEQ ID NO: 9]

This sequence shows the amino acid sequence of the polypeptide of the invention consisting of 9 amino acid residues.
[SEQ ID NO: 10]

This sequence shows the base sequence of the DNA encoding the polypeptide having the amino acid sequence of SEQ ID NO: 9.
[SEQ ID NO: 11]

This sequence shows the amino acid sequence of the polypeptide of the invention consisting of 17 amino acid residues.
[SEQ ID NO: 12]

This sequence shows the base sequence of the DNA encoding the polypeptide having the amino acid sequence of SEQ ID NO: 11.
[SEQ ID NO: 13]

This sequence shows the amino acid sequence of bFGF CS23 mutein.
[SEQ ID NO: 14]

This sequence shows the base sequence of the gene fragment encoding the fusion protein represented by formula (1).
[SEQ ID NO: 15]

This sequence shows the base sequence of primer # 1 used for preparing the structural gene of the polypeptide of the invention in Example 1.
[SEQ ID NO: 16]

This sequence shows the base sequence of primer # 2 used for preparing the structural gene of the polypeptide of the invention in Example 1.
[SEQ ID NO: 17]

This sequence shows the base sequence of primer # 3 used for preparing the structural gene of the polypeptide of the invention in Example 1.
[SEQ ID NO: 18]

This sequence shows the base sequence of primer # 4 used for preparing the structural gene of the polypeptide of the invention in Example 1.
[SEQ ID NO: 19]

This sequence shows the amino acid sequence of the human precursor polypeptide of the invention.
[SEQ ID NO: 20]

This sequence shows the base sequence of the DNA encoding the precursor polypeptide having the amino acid sequence of SEQ ID NO: 19.
[SEQ ID NO: 21]

This sequence shows the amino acid sequence of the human precursor polypeptide of the invention.
[SEQ ID NO: 22]

This sequence shows the base sequence of the DNA encoding the precursor polypeptide having the amino acid sequence of SEQ ID NO: 21.
[SEQ ID NO: 23]

This sequence shows the amino acid sequence of the bovine precursor polypeptide of the invention.
[SEQ ID NO: 24]

This sequence shows the base sequence of the DNA encoding the precursor polypeptide having the amino acid sequence of SEQ ID NO: 23.
[SEQ ID NO: 25]

This sequence shows the amino acid sequence of the mouse precursor polypeptide of the invention.
[SEQ ID NO: 26]

This sequence shows the base sequence of the DNA encoding the precursor polypeptide having the amino acid sequence of SEQ ID NO: 25.
[SEQ ID NO: 27]

This sequence shows the amino acid sequence of the rat precursor polypeptide of the invention.
[SEQ ID NO: 28]

This sequence shows the base sequence of the DNA encoding the precursor polypeptide having the amino acid sequence of SEQ ID NO: 27.
[SEQ ID NO: 29]

This sequence shows the amino acid sequence of the rat precursor polypeptide of the invention.
[SEQ ID NO: 30]

This sequence shows the base sequence of the DNA encoding the precursor polypeptide having the amino acid sequence of SEQ ID NO: 29.

The transformant Escherichia coli MM294 (DE3)/pTFCRFRP-1, obtained in Example 2 described later, is deposited in the International Patent Organism Depositary, the National Institute of Advanced Industrial Science and Technology (now-defunct National Institute of Bioscience and Human Technology (NIBH), Agency of Industrial Science and Technology, Ministry of International Trade and Industry), located at Center No.6, 1-1-1 Higasi, Tukuba-shi, Ibaraki, 305-8566, Japan, under Accession Number FERM BP-7313 as of September 28, 2000; and in the Institute for Fermentation, Osaka (IFO), located at 2-17-85, Jyuso-Honmati, Yodogawa-ku, Osaka-shi, Osaka, 532-8686, Japan, under Accession Number IFO 16476 since September 19, 2000.

The present invention will be described in more detail below with reference to the following examples, which are however not intended to limit the scope of the present invention thereto.

### EXAMPLES

### Example 1: Preparation of the structural gene encoding the polypeptide of the invention (the polypeptide having the amino acid sequence of SEQ ID NO: 7)

The structural gene encoding the polypeptide of the invention (the polypeptide having the amino acid sequence of SEQ ID NO: 7) was prepared using 4 different DNA fragments represented by SEQ ID NOs: 15 to 18 (# 1: SEQ ID NO: 15; # 4: SEQ ID NO: 18, Kikotek Co.)(# 2: SEQ ID NO: 16; # 3: SEQ ID NO: 17, Amersham Pharmacia Biotech Co.) according to a well-known method.

### (a) Phosphorylation of the DNA oligomers

The phosphorylation of 5' terminals of the 2 oligomers, excluding # 1 and # 4, was conducted by incubating 1 µg of each DNA oligomer at 37° C for one hour in 100 µL phosphorylation reaction solution (50 mM Tris-HCl (pH 7.6), 10 mM MgCl₂, 1 mM spermidine, 10 mM dithiothreitol, 0.1 mg/mL bovine serum albumin, 1 mM ATP, and 10 units T4 polynucleotide kinase (Nippon Gene)). After treating the solution with phenol, 2 times vol. of ethanol was added to the aqueous layer recovered. After cooled to -70 °C, the DNA was precipitated by centrifugation.

### (b) Ligation of the DNA fragments

The DNA fragments phosphorylated in (a), # 1 fragment, and # 4 fragment were mixed and added to the solution of 10 mM Tris/HCl and 2 mM EDTA (pH8.0) in a final volume of 120 µl. This mixture was incubated 80 °C for 10 min, and then cooled slowly to a room temperature to achieve annealing. The ligation was conducted using TaKaRa DNA Ligation Kit ver. 2 (Takara) by adding 30 µL of the Kit solution II to 30 µL of the annealing solution, mixing well, adding 60 µL of the Kit solution I, and then incubating at 37 °C for one hour. After treating the solution with phenol, 2 times vol. of ethanol was added to the aqueous layer recovered. After cooled to -70 °C, the DNA was precipitated by centrifugation.

### (c) Phosphorylation of the 5'-terminal

The precipitated DNA was dissolved in 10 µL of TE buffer solution (10 mM Tris-HCl (pH8.0), 1 mM EDTA), and incubated at 37° C for one hour in 100 µL phosphorylation reaction solution (50 mM Tris-HCl (pH 7.6), 10 mM MgCl₂, 1 mM spermidine, 10 mM dithiothreitol, 0.1 mg/mL bovine serum albumin, 1 mM ATP, and 10 units T4 polynucleotide kinase (Nippon Gene)) for the phosphorylation of the 5'-terminal. After treating the solution with phenol, 2 times vol. of ethanol was added to the aqueous layer recovered. After cooled to -70 °C, the DNA was precipitated by centrifugation, and then dissolved in 20 µL of TE buffer solution.

### Example 2: Preparation of the expression plasmid for the polypeptide of the invention (the polypeptide having the amino acid sequence of SEQ ID NO: 7)

To make the expression vector, pTFC (Publication of Patent Application: No.2000-270871) was digested with Ndel and Aval (Takara) at 37° C for 4 hours. After 1% agarose gel electrophoresis, 4.4 kb DNA fragment was extracted using the QIAquick Gel Extraction Kit (QIAGEN) and was dissolved in 25 µL TE buffer solution. This Ndel/Aval fragment derived from pTFC and the structural gene of the polypeptide (having the amino acid sequence of SEQ ID NO: 7) as prepared above were ligated using the TaKaRa DNA Ligation Kit ver. 2 (Takara).

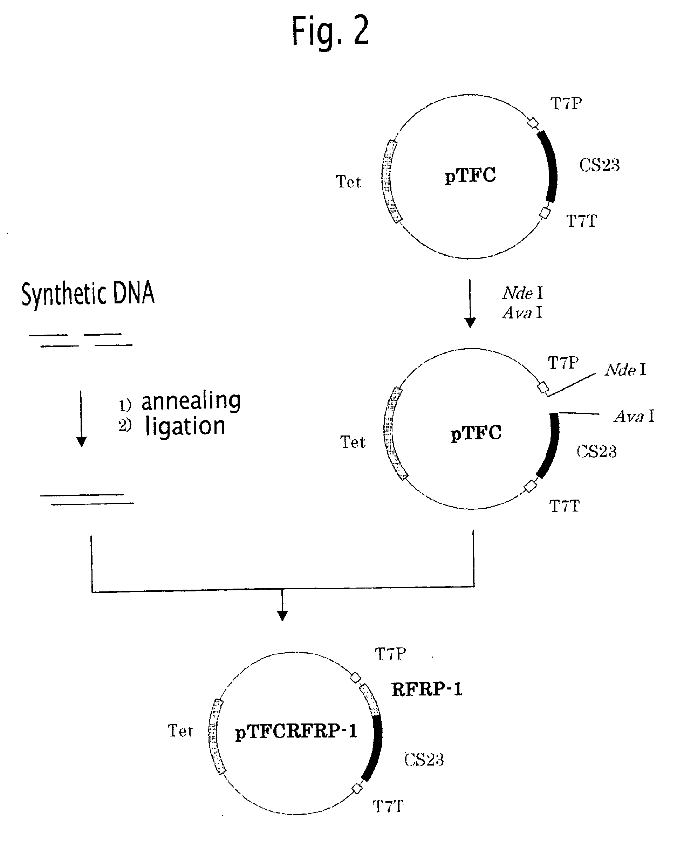

Escherichia coli JM 109 competent cells (Toyobo) were transformed with 10 µL of the ligation solution, inoculated on an LB agar medium containing 10 µg/mL tetracycline. After cultured overnight at 37° C, a tetracycline resistant colony was selected. The transformant was cultured overnight at 37° C in LB medium, and the plasmid pTFCRFRP-1 was prepared from the culture using the QIAprep8 Miniprep Kit (QIAGEN)(Fig. 2). The nucleic acid sequence of the structural gene of the polypeptide (having the amino acid sequence of SEQ ID NO: 7) was confirmed using the Applied Biosystems, Model 377 DNA sequencer. Escherichia coli MM294 (DE3) was transformed with the plasmid pTFCRFRP-1 to obtain a strain of Escherichia coli, M294(DE3)/pTFCRFRP-1 expressing RFRP-1/CS23 fusion protein.

### Example 3:

The transformant obtained in Example 2 was cultured with shaking at 37 °C for 8 hours in a 2-L flask containing 1 L of LB medium (1% peptone, 0.5% yeast extract, 0.5% sodium chloride) containing 5.0 mg/L tetracycline. The culture obtained was transferred to a 50-L fermentor containing 19 L of a main fermentation medium (1.68% sodium monohydrogen phosphate, 0.3% potassium dihydrogen phosphate, 0.1% ammonium chloride, 0.05% sodium chloride, 0.05% magnesium sulfate, 0.02% defoaming agent, 0.00025% ferrous sulfate, 0.00025% thiamin hydrochloride, 1.5% glucose, and 1.5% casamino acid), and then incubated at 30 °C with aeration and agitation. At 500 kleets of the turbidity in the culture, isopropyl-β-D-thiogalactopyranoside was added to the culture at a final concentration of 12 mg/L, and the culture was incubated for another 6 hours. After the culture was completed, approximately 500 g (wet weight) cells were obtained by centrifugation of the culture, and stored in a frozen state at -80 °C.

### Example 4: Production of the polypeptide of the invention (the polypeptide having the amino acid sequence of SEQ ID NO: 7)

To 500 g of the cells obtained in Example 2, 1000 mL of the solution of 6 M guanidine hydrochloride and 0.2 M Tris/HCl (pH 8.0) was added. After shaking for about 4 hrs., the centrifugal separation was conducted (10,000 rpm, 60 min.) to obtain a supernatant, which was diluted with 29 L of 0.6 M arginine, 1 mM dithiothreitol, and 50 mM Tris/HCl (pH 8.0). After an overnight incubation at 10 °C, the supernatant was adjusted to pH 6.0, and passed through an AF-Heparin Toyopearl 650M column (11.3 cm ID x 13.5 cm L, Tosoh) for the absorption, which had been equilibrated with 50 mM phosphate buffer solution (pH 6.0). After the column was washed, the elution was made with 50 mM phosphate buffer solution containing 2M NaCl, pH 6.0 to obtain 1000 mL of the fraction containing the polypeptide (having the amino acid sequence of SEQ ID NO: 7)/CS23 fusion protein.

This effluent was concentrated using the Pellicon Mini Cassette (Millipore) with 0.1 M acetic acid added thereto to obtain the polypeptide (having the amino acid sequence of SEQ ID NO: 7)/CS23 fusion protein in 0.1 M acetic acid solution. After adding urea to the solution at a final concentration of 6 M, 445 mg of 1-cyano-4-diethylaminopyridinium salt (DMAP-CN) was added and incubated for 15 min. at room temperature for the reaction. After completion of the reaction, the reaction solution was passed through a Sephadex G-25 column (46 mm ID x 600 mm L, Pharmacia) equilibrated with 10 % acetic acid. Elution with 10 % acetic acid used for the equilibration at a flow rate of 6 mL/min gave the fraction containing the polypeptide (the polypeptide having the amino acid sequence of SEQ ID NO: 7)/CS23 fusion protein in the S-cyanized form. This effluent was concentrated and desalted using the Pellicon Mini Cassette (Millipore) to obtain the polypeptide (having the amino acid sequence of SEQ ID NO: 7)/CS23 fusion protein in the desalted solution. After adding urea to this desalted solution at a final concentration of 6 M, 25% ammonia water was further added at a final concentration of 3 M, and incubated for 15 min. at 15 °C for the reaction. After the reaction was completed, the solution was adjusted to pH 6.0 with acetic acid to produce the polypeptide (having the amino acid sequence of SEQ ID NO: 7).

This reaction solution was passed through an SP-5PW column (5.5 mm ID x 30 cm L, Tosoh) for the absorption, which had been equilibrated with 50 mM MES buffer solution containing 3 M urea (pH4.5). After the column was washed, the elution was made with a gradient from 0 to 50 % B (B = 50 mM phosphate buffer solution + 1 M NaCI + 3 M urea) to obtain a fraction containing the polypeptide (having the amino acid sequence of SEQ ID NO: 7)(the elution time: 60 min). This polypeptide fraction was further passed through an ODS-120T column (21.5 mm ID x 300 mm L, Tosoh) for the absorption, which had been equilibrated with 0.1 % trifluoroacetate (TFA). After the column was washed, the elution was made with a gradient from 30 to 60 % B (B: 80% acetonitrile / 0.1% TFA). The fractions containing the polypeptide of the invention (having the amino acid sequence of SEQ ID NO: 7)(the elution time: 45 min.) were pooled and lyophilized to obtain the polypeptide of the invention (having the amino acid sequence of SEQ ID NO: 7) as a lyophilized powder.

### (a) Analysis of amino acid composition

The amino acid composition was determined for the obtained polypeptide of the invention (the polypeptide having the amino acid sequence of SEQ ID NO: 7) using an amino acid analyzer (Hitachi L-8500A) (acid hydrolysis with 6N HCl-4% thioglycolic acid at 110 °C for 24, 48 hrs).

The result was consistent with the amino acid composition predicted from the DNA sequence encoding the polypeptide of the invention (the polypeptide having the amino acid sequence of SEQ ID NO: 7) (Table 1).

**Table I**

| Amino acid | Number of residue per mol | Number predicted from the DNA sequence of the present polypeptide |
|---|---|---|
| Asx | 4.2 | 5 |
| Thr¹⁾ | 0.9 | 1 |
| Ser¹⁾ | 2.7 | 3 |
| Glx | 2.0 | 2 |
| Pro | 3.9 | 4 |
| Gly | 1.0 | 1 |
| Ala | 1.9 | 2 |
| Cys²) | | 0 |
| Val | 1.7 | 2 |
| Met | 1.8 | 2 |
| Ile | 0.7 | 1 |
| Leu | 4 | 4 |
| Tyr | 0 | 0 |
| Phe | 2.8 | 3 |
| His | 1.0 | 1 |
| Lys | 3.7 | 4 |
| Arg | 1.0 | 1 |
| Trp | 0.4 | 1 |

| | | |
|---|---|---|
| 1) Value extrapolated at 0 hr. | | |
| 2) not detected | | |

### (b) Analysis of N-terminal amino acid sequence

The N-terminal amino acid sequence was determined for the obtained polypeptide of the invention (the polypeptide having the amino acid sequence of SEQ ID NO: 7) using a gas phase protein sequencer (Applied Biosystems, Model 477A)(100 pmol of the polypeptide of the invention (the polypeptide having the amino acid sequence of SEQ ID NO: 7) was used for analysis). The result show that the determined N-terminal amino acid sequence was identical to the N-terminal amino acid sequence predicted from the DNA sequence (Table 2).

**Table 2**

| Residue No. | PTH¹⁾-amino acid detected (pmol) | Amino acid predicted from the base sequence of the present polypeptide |
|---|---|---|
| 1 | Ser (47) | Ser |
| 2 | Leu (58) | Leu |
| 3 | Asn (63) | Asn |
| 4 | Phe (66) | Phe |
| 5 | Glu (55) | Glu |
| 6 | Glu (55) | Glu |
| 7 | Leu (50) | Leu |
| 8 | Lys (60) | Lys |
| 9 | Asp (48) | Asp |
| 10 | Trp(18) | Trp |
| 11 | Gly(37) | Gly |
| 12 | Pro (26) | Pro |
| 13 | Lys (48) | Lys |
| 14 | Asn (27) | Asn |
| 15 | Val (28) | Val |
| 16 | Ile (25) | Ile |
| 17 | Lys (46) | Lys |
| 18 | Met (28) | Met |
| 19 | Ser(12) | Ser |
| 20 | Thr (11) | Thr |

| | | |
|---|---|---|
| 1) Phenylthiohydantoin | | |

### (c) Analysis of C-terminal amino acid

The C-terminal amino acid was analyzed for the obtained polypeptide of the invention (the polypeptide having the amino acid sequence of SEQ ID NO: 7) using an amino acid analyzer (Hitachi L-8500A), but there was no detection because the C-terminal was amidated.

**Table 3:**

| Analysis of C-terminal amino acid | | |
|---|---|---|
| | C-terminal amino acid | Recovery (%) |
| RFRP-1 | Phe | - |
| Gas phase hydrazinolysis (100°C, 6 hrs) | | |

### Example 5:

The activity of inhibiting the cAMP production was determined for the polypeptide of the invention obtained in Example 4 (the polypeptide having the amino acid sequence of SEQ ID NO: 7) according to the method described in Example 1 of WO 00/29441. As a result, it was confirmed that the said polypeptide has the activity as much as the synthetic polypeptide (the polypeptide having the amino acid sequence of SEQ ID NO: 7).

### INDUSTRIAL APPLICABILITY

Using the production method of the present invention, it is possible to produce industrially in a large scale the polypeptide which can be used as a prophylactic or therapeutic agent for various diseases associated with hypo-secretion or hyper-secretion of prolactin.

## Claims

1. A method of producing a partial peptide of the polypeptide comprising the amino acid sequence of SEQ ID NO: 19, 21, 23, 25, 27 or 29, or a salt thereof, which comprises:
subjecting a fusion protein or polypeptide, in which a partial peptide of the polypeptide comprising the amino acid sequence of SEQ ID NO: 19, 21, 23, 25, 27 or 29, which may has a methionine residue optionally oxidized at the N-terminal, is ligated to the N-terminal of a protein or polypeptide having a cysteine at the N-terminal, to a reaction for cleavage of a peptide bond on the amino group side of the cysteine residue.

2. A method of producing a partial peptide of the polypeptide comprising the amino acid sequence of SEQ ID NO: 19, 21, 23, 25, 27 or 29, which may has a methionine residue at the N-terminal, or a salt thereof, which comprises:
culturing a transformant containing a vector having a DNA encoding a fusion protein or polypeptide, in which the partial peptide of the polypeptide comprising the amino acid sequence of SEQ ID NO: 19, 21, 23, 25, 27 or 29, which may has a methionine residue at the N-terminal, is ligated to the N-terminal of a protein or polypeptide having a cysteine at the N-terminal to express the fusion protein or polypeptide; and
subjecting the expressed fusion protein or polypeptide to a reaction for cleavage of a peptide bond on the amino group side of the cysteine residue.

3. The production method of claim 1 or 2, wherein the cleavage reaction is S-cyanation reaction followed by an ammonolysis or hydrolysis reaction.

4. The production method of claim 1 or 2, wherein the partial peptide of the polypeptide comprising the amino acid sequence of SEQ ID NO: 19, 21, 23, 25, 27 or 29 includes:
(i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
(ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
(iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
(iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
(v) a peptide comprising the amino acid sequence from aa 58 (Ser) to aa 94 (Phe), or aa 84 (Pro) to aa 94 (Phe) in the amino acid sequence of SEQ ID NO: 27.

5. The production method of claim 1 or 2, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.

6. The production method of claim 5, wherein the polypeptide comprising the amino acid sequence of SEQ ID NO: 1 or 9 includes the polypeptide having the amino acid sequence of SEQ ID NO: 1, 3, 5, 7, 9 or 11.

7. A fusion protein or polypeptide, in which a partial peptide of the polypeptide comprising the amino acid sequence of SEQ ID NO: 19, 21, 23, 25, 27 or 29, which may has a methionine residue at the N-terminal, is ligated to the N-terminal of a protein or polypeptide having a cysteine at the N-terminal, or a salt thereof.

8. The polypeptide of claim 7 or a salt thereof, wherein the partial peptide of the polypeptide comprising the amino acid sequence of SEQ ID NO: 19, 21, 23, 25, 27 or 29 includes:
(i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Set), aa 95 (Asn) to aa 112 (Ser), aa 115 (Asn) to aa 131 (Phe), aa 124 (Val) to aa 131 (Pile), aa 1 (Met) to aa 92 (Phe), aa 1 (Met) to aa 112 (Ser), or aa 1 (Met) to aa 131 (Phe) in the amino acid sequence of SEQ ID NO: 19;
(ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
(iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 1 12 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
(iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
(v) a peptide comprising the amino acid sequence from aa 58 (Ser) to aa 94 (Phe), or aa 84 (Pro) to aa 94 (Phe) in the amino acid sequence of SEQ ID NO: 27.

9. The polypeptide of claim 7 or a salt thereof, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.

10. The polypeptide of claim 9 or a salt thereof, wherein the polypeptide comprising the amino acid sequence of SEQ ID NO: 1 or 9 includes the polypeptide having the amino acid sequence of SEQ ID NO: 1, 3, 5, 7, 9 or 11.

11. A vector comprising the DNA encoding the fusion protein or polypeptide of claim 7.

12. A transformant having the vector of claim 11.

13. A method of removing the N-terminal methionine residue from a partial peptide of the polypeptide comprising the amino acid sequence of SEQ ID NO: 19, 21, 23, 25, 27 or 29, which has a methionine residue optionally oxidized at the N-terminal, which comprises:
reacting the partial peptide or a salt thereof with α-diketone; and then
subjecting it to a hydrolysis reaction.

14. The method of claim 13, wherein the partial peptide of the polypeptide comprising the amino acid sequence of SEQ ID NO: 19, 21, 23, 25, 27 or 29, which has a methionine residue optionally oxidized at the N-terminal, is produced by genetic engineering, and the amino acid next to the methionine residue on the C-terminal side is Isoleucine, Valine, Cysteine, Threonine, Aspartic acid, Lysine, Leucine, Arginine, Asparagine, Methionine, Phenylalanine, Tyrosine, Tryptophan, Glutamic acid, Glutamine or Histidine.

15. The method of claim 13 or 14, wherein the partial peptide of the polypeptide comprising the amino acid sequence of SEQ ID NO: 19, 21, 23, 25, 27 or 29 includes:
(i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
(ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Pile), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
(iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
(iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
(v) a peptide comprising the amino acid sequence from aa 58 (Ser) to aa 94 (Phe), or aa 84 (Pro) to aa 94 (Phe) in the amino acid sequence of SEQ ID NO: 27.

16. The method of claim 13 or 14, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.

17. The method of claim 16, wherein the polypeptide comprising the amino acid sequence of SEQ ID NO: 1 or 9 includes the polypeptide having the amino acid sequence of SEQ ID NO: 1,3, 5, 7, 9 or 11.

18. The method of claim 13, wherein the reaction with α-diketone is carried out in the presence of a transition metal ion.

19. The method of claim 13, wherein the reaction with α-diketone is carried out in the presence of a base.

20. The method of claim 13, wherein the reaction with α-diketone is carried out in the presence of a transition metal ion and a base.

21. The method of claim 13, wherein the α-diketone is glyoxylic acid or its salt.

22. The method of claim 18, wherein the transition metal ion is a copper ion.

23. The method of claim 19, wherein the base is pyridine.

24. The method of claim 13, wherein the hydrolysis reaction is carried out using a base.

25. The method of claim 24, wherein the base is an amine.

26. The method of claim 24, wherein the base is a diamine, or thio- or selenosemicarbazide.

27. The method of claim 26, wherein the diamine is o-phenylenediamine or 3,4-diamino-benzoic acid.

28. A method of producing the polypeptide comprising the amino acid sequence of SEQ ID NO: 1 or 9, or a salt thereof, which comprises:
reacting a partial peptide of the polypeptide comprising the amino acid sequence of SEQ ID NO: 19, 21, 23, 25, 27 or 29, which has a methionine residue added at the N-terminal, or a salt thereof, which is produced by genetic engineering, with glyoxylic acid or its salt in the presence of copper sulfate and pyridine; and then
reacting it with o-phenylenediamine or 3,4-diamino-benzoic acid.

29. The production method of claim 28, wherein the partial peptide of the polypeptide comprising the amino acid sequence of SEQ ID NO: 19, 21, 23, 25, 27 or 29 includes:
(i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
(ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
(iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
(iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
(v) a peptide comprising the amino acid sequence from aa 58 (Ser) to aa 94 (Phe), or aa 84 (Pro) to aa 94 (Phe) in the amino acid sequence of SEQ ID NO: 27.

30. The production method of claim 28, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.

31. The production method of claim 30, wherein the polypeptide comprising the amino acid sequence of SEQ ID NO: 1 or 9 includes the polypeptide having the amino acid sequence of SEQ ID NO: 1, 3, 5, 7, 9 or 11.

32. A compound represented by the formula CH₃-S(O)ₘ-(CH₂)₂-CO-CO-X, or a salt thereof, wherein m indicates an integer of 0 to 2, and X indicates a partial peptide chain of the polypeptide comprising the amino acid sequence of SEQ ID NO: 19, 21, 23, 25, 27 or 29.

33. The compound of claim 32, or a salt thereof, wherein the partial peptide of the polypeptide comprising the amino acid sequence of SEQ ID NO: 19, 21, 23, 25, 27 or 29 includes:
(i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
(ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser) or aa 1 (Met) to aa 131 (Phe) in the amino acid sequence of SEQ ID NO: 21;
(iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
(iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
(v) a peptide comprising the amino acid sequence from aa 58 (Ser) to aa 94 (Phe), or aa 84 (Pro) to aa 94 (Phe) in the amino acid sequence of SEQ ID NO: 27.

34. The compound of claim 32, or a salt thereof, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.

35. The compound of claim 34, or a salt thereof, wherein the polypeptide comprising the amino acid sequence of SEQ ID NO: 1 or 9 includes the polypeptide having the amino acid sequence of SEQ ID NO: 1, 3, 5, 7, 9 or 11.

36. A method of producing a partial peptide of the polypeptide comprising the amino acid sequence of SEQ ID NO: 19, 21, 23, 25, 27 or 29, or a salt thereof, which comprises subjecting the compound of claim 32 to a hydrolysis reaction.

37. A method of producing the polypeptide comprising the amino acid sequence represented by , or a salt thereof, which comprises:
subjecting a fusion protein or polypeptide, in which the polypeptide comprising the amino acid sequence represented by , which has a methionine residue at the N-terminal, is ligated to the N-terminal of a protein or polypeptide having a cysteine at the N-terminal, to a reaction for cleavage of a peptide bond on the amino group side of the cysteine residue to obtain the polypeptide comprising the amino acid sequence represented by , which has a methionine residue optionally oxidized at the N-terminal, or a salt thereof;
reacting the polypeptide comprising the amino acid sequence represented by , which has a methionine residue optionally oxidized at the N-terminal, or a salt thereof with α-diketone; and
subjecting it to a hydrolysis reaction.

38. The production method of claim 37, wherein the partial peptide of the polypeptide comprising the amino acid sequence of SEQ ID NO: 19, 21, 23, 25, 27 or 29 includes:
(i) a peptide comprising the amino acid sequence from aa 56 (Ser) to aa 92 (Phe), aa 73 (Met) to aa 92 (Phe), aa 81 (Met) to aa 92(Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 112 (Ser), or aa 1 (Met) to aa 131 (Phe) in the amino acid sequence of SEQ ID NO: 19;
(ii) a peptide comprising the amino acid sequence from aa 56 (Ser) to aa 92 (Phe), aa 73 (Met) to aa 92 (Phe), aa 81 (Met) to aa 92 (Phe), aa 84 (Ser) to aa 92 (Phe), aa 101 (Ser) to aa 112 (Ser), aa 95 (Asn) to aa 112 (Ser), aa 115 (Asn) to aa 131 (Phe), aa 124 (Val) to aa 131 (Phe), aa 1 (Met) to aa 92 (Phe), aa 1 (Met) to aa 12 (Ser) or aa 1 (Met) to aa 131 (Phe) in the amino acid sequence of SEQ ID NO: 21;
(iii) a peptide comprising the amino acid sequence from aa 58 (Ser) to aa 92 (Phe), aa 81 (Met) to aa 92 (Phe), aa 101 (Ser) to aa 112 (Leu), aa 95 (Asn) to aa 112 (Leu), aa 124 (Val) to aa 131 (Phe), aa 1 (Met) to aa 92 (Phe), or aa 1 (Met) to aa 131 (Phe) in the amino acid sequence of SEQ ID NO: 23;
(iv) a peptide comprising the amino acid sequence from aa 58 (Ser) to aa 94 (Phe), aa 83 (Val) to aa 94 (Phe), aa 84 (Pro) to aa 94 (Phe), or aa 118 (Phe) to aa 125 (Phe) in the amino acid sequence of SEQ ID NO: 25; or
(v) a peptide comprising the amino acid sequence from aa 58 (Ser) to aa 94 (Phe), or aa 84 (Pro) to aa 94 (Phe) in the amino acid sequence of SEQ ID NO: 27.

39. The production method of claim 37, wherein the partial peptide of the polypeptide comprising the amino acid sequence of SEQ ID NO: 19, 21, 23, 25, 27 or 29 includes the polypeptide comprising the amino acid sequence of SEQ ID NO: 1 or 9.

40. The production method of claim 39, wherein the polypeptide comprising the amino acid sequence of SEQ ID NO: 1 or 9 includes the polypeptide having the amino acid sequence of SEQ ID NO: 1, 3, 5, 7, 9 or 11.
